(19) Europäisches Patentamt
European Patent Office
Office européen des brevets

(11) **EP 2 741 225 A2**

(12) **EUROPEAN PATENT APPLICATION**

(43) Date of publication:
**11.06.2014 Bulletin 2014/24**

(51) Int Cl.:
***G06F 19/00*** *(2011.01)*

(21) Application number: **13193511.6**

(22) Date of filing: **19.11.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **20.11.2012 US 201213682443**

(71) Applicant: **Thermo Finnigan LLC**
**San Jose, CA 95134 (US)**

(72) Inventor: **Wright, David A.**
**Livermore, CA 94550 (US)**

(74) Representative: **Boult Wade Tennant**
**Verulam Gardens**
**70 Gray's Inn Road**
**London WC1X 8BT (GB)**

(54) **Automatic reconstruction of MS-2 spectra from all-ions-fragmentation to recognize previously detected compounds**

(57) A method of acquiring and interpreting data using a mass spectrometer system and a local mass spectral library comprises: (a) generating a multiplexed mass spectrum, the multiplexed mass spectrum comprising a superposition of a plurality of product-ion mass spectra comprising a plurality of product-ion types, each product-ion mass spectrum corresponding to fragmentation of a respective precursor-ion type; (b) recognizing a respective set of two or more product-ion types corresponding to each of one or more of the product-ion mass spectra by recognizing correlations between the elution profiles of said two or more product-ion types corresponding to each said respective set; and (c) determining if each recognized set of two or more product-ion types corresponds to a product-ion mass spectrum previously observed using said mass spectrometer system by comparing the $m/z$ ratios of the product ion types to information in at least one entry of the local mass spectral library.

FIG. 5

EP 2 741 225 A2

**Description**

FIELD OF THE INVENTION

This invention relates to methods of analyzing data obtained from instrumental analysis techniques used in analytical chemistry and, in particular, to methods of automatically analyzing and storing, in a local mass spectral library, mass spectral data generated in LC/MS/MS analyses that do not include a precursor ion selection step.

BACKGROUND OF THE INVENTION

Mass spectrometry (MS) is an analytical technique to filter, detect, identify and/or measure compounds by the mass-to-charge ratios of ions formed from the compounds. The quantity of mass-to-charge ratio is commonly denoted by the symbol "*m/z*" in which "*m*" is ionic mass in units of Daltons and "*z*" is ionic charge in units of elementary charge, e. Thus, mass-to-charge ratios are appropriately measured in units of "Da/e". Mass spectrometry techniques generally include (1) ionization of compounds and optional fragmentation of the resulting ions so as to form fragment ions; and (2) detection and analysis of the mass-to-charge ratios of the ions and/or fragment ions and calculation of corresponding ionic masses. The compound may be ionized and detected by any suitable means. A "mass spectrometer" generally includes an ionizer and an ion detector.

One can often enhance the resolution of the MS technique by employing "tandem mass spectrometry" or "MS/MS", for example via use of a triple quadrupole mass spectrometer. In this technique, a first, or parent, or precursor, ion generated from a molecule of interest can be filtered or isolated in an MS instrument, and these precursor ions subsequently fragmented to yield one or more second, or product, or fragment, ions that are then analyzed in a second MS stage. By careful selection of precursor ions, only ions produced by certain analytes are passed to the fragmentation chamber or other reaction cell, such as a collision cell where collision of ions with atoms of an inert gas produces the fragment ions. Because both the precursor and fragment ions are produced in a reproducible fashion under a given set of ionization/fragmentation conditions, the MS/MS technique can provide an extremely powerful analytical tool. For example, the combination of precursor ion selection and subsequent fragmentation and analysis can be used to eliminate interfering substances, and can be particularly useful in complex samples, such as biological samples. Selective reaction monitoring (SRM) is one commonly employed tandem mass spectrometry technique.

The hybrid technique of liquid chromatography-mass spectrometry (LC/MS) is an extremely useful technique for detection, identification and (or) quantification of components of mixtures or of analytes within mixtures. This technique generally provides data in the form of a mass chromatogram, in which detected ion intensity (a measure of the number of detected ions) as measured by a mass spectrometer is given as a function of time. In the LC/MS technique, various separated chemical constituents elute from a chromatographic column as a function of time. As these constituents come off the column, they are submitted for mass analysis by a mass spectrometer. The mass spectrometer accordingly generates, in real time, detected relative ion abundance data for ions produced from each eluting analyte, in turn. Thus, such data is inherently three-dimensional, comprising the two independent variables of time and mass (more specifically, a mass-related variable, such as mass-to-charge ratio) and a measured dependent variable relating to ion abundance.

Generally, "liquid chromatography" (LC) means a process of selective retention of one or more components of a fluid solution as the fluid uniformly percolates through a column of a finely divided substance, or through capillary passageways. The retention results from the distribution of the components of the mixture between one or more stationary phases and the bulk fluid, (i.e., mobile phase), as this fluid moves relative to the stationary phase(s). "Liquid chromatography" includes, without limitation, reverse phase liquid chromatography (RPLC), high performance liquid chromatography (HPLC), ultra high performance liquid chromatography (UHPLC), supercritical fluid chromatography (SFC) and ion chromatography.

Recent improvements in liquid chromatography (LC) throughput and mass spectrometry (MS) detection capabilities have led to a surge in the use of LC/MS-based techniques for screening, confirmation and quantification of ultra-trace levels of analytes. There is currently a trend towards full-scan MS experiments in residue analysis. Such full-scan approaches utilize high performance time-of-flight (TOF) or electrostatic trap (such as Orbitrap®-type) mass spectrometers coupled to UHPLC columns and can facilitate rapid and sensitive screening and detection of analytes. The superior resolving power of the Orbitrap® mass spectrometer (up to 100,000 FWHM) compared to TOF instruments (10,000-20,000) ensures the high mass accuracy required for complex sample analysis.

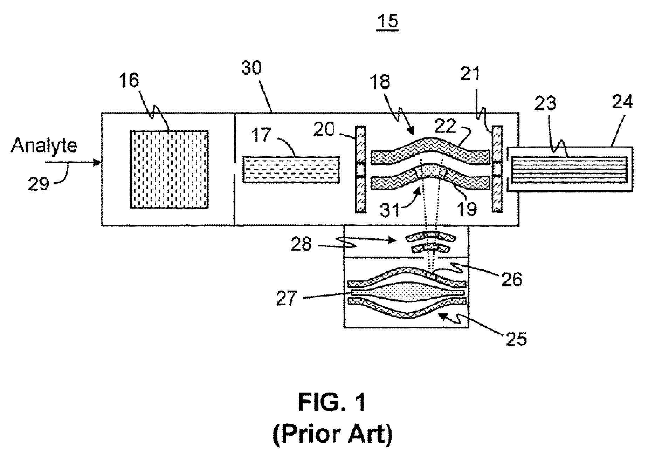

An example of a mass spectrometer system **15** comprising an electrostatic trap mass analyzer such as an Orbitrap® mass analyzer **25** is shown in FIG. 1. Analyte material **29** is provided to a pulsed or continuous ion source **16** so as to generate ions. Ion source **16** could be a MALDI source, an electrospray source or any other type of ion source. In addition, multiple ion sources may be used. The illustrated system comprises a curved quadrupole trap **18** (also known as a "C-trap") with a slot **31** in the inner electrode **19.** Ions are transferred from the ion source **16** to the curved quadrupole trap **18** by ion optics assembly **17** (e.g. an RF multipole). Prior to ion injection, ions may be squeezed along the axis of the curved quadrupole trap **18** by raising voltages on end electrodes **20** and **21.** For ion injection into the Orbitrap® mass analyzer **25,** the RF voltage on the curved quadrupole trap **18** may be switched off, as is well known. Pulses are applied to electrodes **19** and **22** and to an electrode of curved ion optics **28** so that the transverse electric field accelerates ions into the curved ion optics **28**. The converging ion beam that results enters the Orbitrap® mass analyzer **25** through injection slot **26.** The ion beam is squeezed towards the axis by an increasing voltage on a central electrode **27.** Due to temporal and spatial focusing at the injection slot **26,** ions start coherent axial oscillations. These oscillations produce image currents that are amplified and processed. Further details of the electrostatic trap apparatus **25** are described in International Application Publication WO 02/078046, US Pat. No. 5,886,346, US Pat. No. 6,872,938. The ion optics assembly **17,** curved quadrupole trap **18** and associated ion optics are enclosed in a housing **30** which is evacuated in operation of the system.

The system **15** (FIG. 1) further comprises reaction cell **23,** which may comprise a collision cell (such as an octopole) that is enclosed in a gas tight shroud **24** and that is aligned to the curved quadrupole trap **141.** The reaction cell **23,** when used as a collision cell, may be supplied with an RF voltage of which the DC offset can be varied. A collision gas line (not shown) may be attached and the cell is pressurized with nitrogen (or any) gas.

Higher energy collisions (HCD) may take place in the system **15** as follows: Ions are transferred to the curved quadrupole trap **18.** The curved quadrupole trap is held at ground potential. For HCD, ions are emitted from the curved quadrupole trap **18** to the octopole of the reaction cell **23** by setting a voltage on a trap lens. Ions collide with the gas in the reaction cell **23** at an experimentally variable energy which may be represented as a relative energy depending on the ion mass, charge, and also the nature of the collision gas (i.e., a normalized collision energy). Thereafter, the fragment ions are transferred from the reaction cell back to the curved quadrupole trap by raising the potential of the octopole. A short time delay (for instance 30 ms) is used to ensure that all of the ions are transferred. In the final step, ions are ejected from the curved quadrupole trap **18** into the Orbitrap® analyzer **25** as described previously.

The mass spectrometer system **15** illustrated in FIG. 1 lacks a mass filtering step and, instead, causes fragmentation of all precursor ions at once, without first selecting particular precursor ions to fragment. Accordingly, the equivalent of a tandem mass spectrometry experiment is performed as follows: (a) a first sample of ions (comprising a plurality of types of ions) produced from an eluting chemical compound are transferred to and captured by the curved quadrupole trap **18;** (b) the first sample of ions is transferred to the Orbitrap® analyzer **25** as described above for analysis, thereby producing a "full-scan" of the ions; (c) after the first sample of ions has been emptied from the curved quadrupole trap **18,** a second sample of ions from the same chemical compound are transferred through the curved quadrupole trap **18** to the reaction cell **23;** (d) in the reaction cell, a plurality of different types of fragment ions are formed from each of the plurality of ion types of the second sample of the chemical compound; (e) once the Orbitrap® analyzer **25** has been purged of the first sample of ions, the fragment ions are transferred back quadrupole trap **18** and then to the Orbitrap® analyzer **25** for analysis as described above. Such "all-ions-fragmentation" (AIF) provides a potential multiplexing advantage, but only if the analysis firmware or software can successfully extract precursor-product relationships between the thousands of ions generated in the all-ions-fragmentation scan and the additional thousands of ions present in the full-MS precursor scan.

The spectrometer system **15** illustrated in FIG. 1 is merely a single example of a mass spectrometer system in accordance with the present teachings, or in conjunction with which methods in accordance with the present teachings may be employed. The present teachings may also be employed in conjunction with other mass spectrometer systems having sufficiently high mass precision and resolution - such as time-of-flight (TOF) and other mass spectrometer systems -if those systems are used for all-ions-fragmentation experiments.

It would be a very powerful feature of a mass spectrometer if it could automatically recognize, in real time, that a sample just run contains many of the same compounds as a sample run at a previous time. The information relating to the compounds previously observed on the mass spectrometer would be stored in a database that may be referred to as a "local spectral library". Unfortunately, however, a simplistic approach to generation of such a library has serious problems both with storage and retrieval. For example, a mass spectrometer of the type illustrated in FIG. 1 is capable of acquiring high-resolution product ion spectra at a rate of 10Hz. Thus, the number of spectra acquired in an hour is $10 \times 60 \times 60 = 36,000$; the number of spectra that may be acquired in a day is $36,000 \times 24 = 864,000$. Thus, an instrument of the type illustrated in FIG. 1 is capable of generating data at a rate of 10 megabytes (MB) per minute or more. Thus, even a 1 terabyte (TB) storage device, such a hard drive, will only be able to contain a few weeks of data if the entire raw files are saved. The process of searching through a terabyte of data for a match cannot be done in the few seconds in which a compound is eluting from the column. Manual curation of the data is very tedious and not possible for real-time analysis, or even any sort of rapid analysis; the instrument produces data thousands of times faster than a trained spectrometrist can analyze it.

Although the total number of product ion spectra that may be obtained over the lifetime of a mass spectrometer may number in the billions - i.e. a million a day for thousands of days -the size of the local mass spectral library depends only upon the number of unique precursors that are detected by the instrument. The number of unique and well characterized molecules recorded by a mass spectrometer is even fewer - typically several orders of magnitude smaller than the total number of molecules. If a database contains one million product ion spectra and each spectrum requires a kilobyte of storage (i.e. four bytes for mass and four bytes of intensity for a few dozen peaks plus annotation), the memory required to store the local spectral library is on the order of one gigabyte (GB). Thus, typical databases that encapsulate a complete record of every precursor a mass spectrometer will ever encounter can be stored locally and accessed rapidly.

SUMMARY

A method of acquisition and analysis of All Ions Fragmentation data is described which can be performed as the data is acquired, or later, in which noise-free automatically reconstructed tandem mass spectra (MS-2 spectra) are automatically generated and compared against an database of previously found spectra determine that the compounds present in the current sample were previously detected.

In order to provide a solution to the problems relating to the size of the mass spectral library, the present teachings describe an automatic procedure to process the large (10-1000 MB) raw data files and extract only the well-characterized MS-2 spectra, so that matches with historical data from the same, or similar instruments, are unambiguous. For an accurate-mass instrument like that shown in FIG. 1, the mass accuracy is in the part-per-million (ppm) range. With such a level of accuracy, a precursor mass and an MS-2 spectrum is often sufficient to unambiguously identify the compound when coupled with a fragment predictor and a search of a routine commercial database that indentifies compounds based on their MS-2 spectra.

According to first aspect of the invention, there is provided a method of acquiring and interpreting data using a mass spectrometer system and a local mass spectral library, the local mass spectral library having a plurality of library entries derived from data previously obtained using said mass spectrometer system, comprising: (a) generating a multiplexed mass spectrum using the mass spectrometer system, the multiplexed mass spectrum comprising a superposition of a plurality of product-ion mass spectra comprising a plurality of product-ion types, each product-ion mass spectrum corresponding to fragmentation of a respective precursor-ion type; (b) recognizing a respective set of product-ion types corresponding to each of one or more of the product-ion mass spectra by recognizing correlations between the elution profiles of said product-ion types of each said respective set; and (c) determining if each recognized set of more product-ion types corresponds to a product-ion mass spectrum previously observed using said mass spectrometer system by comparing the $m/z$ ratios of the product ion types of each said recognized set to at least one entry of the mass spectral library.

According to a second aspect of the invention, there is provided a method of acquiring and interpreting data using a mass spectrometer system and a local mass spectral library, the local mass spectral library having a plurality of library entries derived from data previously obtained using said mass spectrometer system, comprising: (a) generating a multiplexed mass spectrum using the mass spectrometer system, the multiplexed mass spectrum comprising a superposition of a plurality of product-ion mass spectra comprising a plurality of product-ion types having respective product-ion mass-to-charge ($m/z$) ratios, each product-ion mass spectrum corresponding to fragmentation of a respective precursor-ion type formed by ionization of a chemical compound, each precursor-ion type having a respective precursor-ion mass-to-charge ($m/z$) ratio; (b) recognizing a set comprising a precursor-ion type and one or more product-ion types corresponding to each of one or more product-ion mass spectra by recognizing one or more losses of a respective valid neutral molecule from each said precursor-ion type; and (c) determining if each recognized set of a precursor-ion type and one or more product-ion types corresponds to a compound whose mass spectra were previously observed using said mass spectrometer system by comparing the $m/z$ ratios of said precursor-ion type and said one or more product ion types of each said recognized set to at least one entry of the mass spectral library.

According to a third aspect of the invention, there is disclosed a method of reducing a size of a computer file of mass spectral data obtained with regard to a sample using a mass spectrometer system, said mass spectral data comprising a plurality of multiplexed mass spectra obtained at respective elution times, wherein each said multiplexed mass spectrum comprises a superposition of a plurality of product-ion mass spectra comprising a plurality of product-ion types, each product-ion mass spectrum corresponding to fragmentation of a respective precursor-ion type formed by ionization of a chemical compound of the sample, each precursor-ion type having a respective precursor-ion mass-to-charge ($m/z$) ratio and each product ion type having a respective product-ion $m/z$ ratio, said method comprising: (a) extracting a respective elution profile of each product-ion type; (b) calculating a respective correlation score between each possible pair of extracted elution profiles; (c) recognizing sets of correlated product-ion types such that the calculated correlation scores between each pair of product-ion types of the set is above a threshold correlation score; and (d) retaining information within the computer file only in regard to those recognized sets for which the number of correlated product-ion types of the set is above a threshold number of product-ion types.

According to another aspect of the invention, there is disclosed a method of reducing a size of a computer file of mass spectral data obtained with regard to a sample using a mass spectrometer system, said mass spectral data comprising a plurality of multiplexed mass spectra obtained at respective elution times, wherein each said multiplexed mass spectrum comprises a superposition of a plurality of product-ion mass spectra comprising a plurality of product-ion types, each product-ion mass spectrum corresponding to fragmentation of a respective precursor-ion type formed by ionization of a chemical compound of the sample, each precursor-ion type having a respective precursor-ion mass-to-charge (*m/z*) ratio and each product ion type having a respective product-ion *m/z* ratio, said method comprising: (a) recognizing a plurality of sets, each set comprising a precursor-ion type and one or more product-ion types such that each product-ion type of each set corresponds to a loss of a respective valid neutral molecule from the precursor-ion type of said each set; and (d) retaining information within the computer file only in regard to those recognized sets for which the number of product-ion types of the set is above a threshold number of product-ion types.

In some embodiments, the mass spectrometer system may include a time-of-flight (TOF) mass analyzer. In various embodiments, the mass spectrometer system may include an electrostatic trap mass analyzer.

BRIEF DESCRIPTION OF THE DRAWINGS

The above noted and various other aspects of the present invention will become apparent from the following description which is given by way of example only and with reference to the accompanying drawings, not drawn to scale, in which:

FIG. 1 is a schematic illustration of an example of a mass spectrometer system comprising an electrostatic trap mass analyzer such as an Orbitrap® mass analyzer;

FIG. 2 is a schematic diagram of a system for generating and automatically analyzing chromatography / mass spectrometry spectra in accordance with the present teachings;

FIG. 3 is a perspective view of a three-dimensional graph of chromatography-mass spectrometry data, in which the variables are time, mass (or mass-to-charge ratio, *m/z*) and ion abundance;

FIG. 4A is a perspective view of a three-dimensional graph of chromatography-mass spectrometry data showing four hypothetical mass spectra of precursor ions and corresponding mass spectra of fragment ions and showing hypothetical extracted ion chromatograms (XICs) for several different values of mass-to-charge ratio;

FIG. 4B is a perspective view of a portion of the three-dimensional graph of FIG. 15A showing selected peaks as extracted ion chromatograms;

FIG. 4C is a perspective view of a portion of the three-dimensional graph of FIG. 15A showing selected peaks as mass scans;

FIG. 5 is a flow chart of a general method for handling mass spectral data in accordance with the present teachings;

FIGS. 6A-6B provide a flowchart of a first method for generating automated correlations between all-ions precursor ions and all-ions-fragmentation product ions in accordance with the present teachings;

FIG. 7A is a flowchart of a method for automated spectral peak detection and quantification in accordance with an embodiment of the present teachings;

FIG. 7B is a schematic example of decomposing a complexly shaped chromatogram trace into resolved peaks;

FIG. 8 is a set of plots of several observed line shapes in various extracted ion chromatograms obtained from LC/MS data covering the 1.7-second elution of a single mass chromatographic peak of a 500 nM solution of the drug Buspirone;

FIG. 9 is a schematic illustration of two peaks having differing line shapes illustrating a method of calculating a cross-correlation score as a dot product;

FIGS. 10A-10B provide a flowchart of a second method for generating automated correlations between all-ions precursor ions and all-ions-fragmentation product ions in accordance with the present teachings;

FIG. 11A is a flow chart of a general method in accordance with the present teachings for acquiring or reading mass spectral data and then interpreting or storing the data or compressing the file size of stored data; and

FIG. 11B is a flow chart of a second general method in accordance with the present teachings for acquiring or reading mass spectral data and then interpreting or storing the data or compressing the file size of stored data.

DETAILED DESCRIPTION

The teachings of the present disclosure are applicable for acquiring data on a mass spectrometer system and interpreting or recognizing that data, as it is acquired, in regard to a local mass spectral library. The present teachings are also applicable to storing the acquired data in the local mass spectral library if the interpretation concludes that the data corresponds to a mass spectrum not previously observed by the mass spectrometer system. The present teachings are further applicable to compressing the size of a file comprising raw, unfiltered data obtained by the mass spectrometer system.

The present disclosure uses the elsewhere-disclosed methods of decomposing superimposed MS-2 spectra obtained from All Ions Fragmentation data by either lineshape correlation or neutral loss correlation. The methods of decomposing spectra according to lineshape correlation are taught in co-pending United States Patent Application No.

12/970,570 filed on January 4 2011 and titled "Method and Apparatus for Correlating Precursor and Product Ions in All-Ions Fragmentation Experiments", said application published as US Publ. No. 2012/0158318 A1 and assigned to the assignee of the present application. The methods of decomposing spectra according to neutral loss correlation are taught in a co-pending United States Patent Application "Use of Neutral Loss Mass to Reconstruct MS-2 Spectra in All-Ions Fragmentation", attorney docket no. 8896US1/NAT, said application filed on even date herewith and assigned to the assignee of the present application.

In referencing the elsewhere-disclosed methods, the present disclosure makes use of the terms "ion" (or "ions" in the plural) and "ion type" (or "ion types" in the plural). For purposes of this disclosure, an "ion" is considered to be a single, solitary charged particle, without implied restriction based on chemical composition, mass, charge state, mass-to-charge ($m/z$) ratio, etc. A plurality of such charged particles comprises a collection of "ions". An "ion type", as used herein, refers to a category of ions - specifically, those ions having a given monoisotopic $m/z$ ratio - and, most generally, includes a plurality of charged particles, all having the same monoisotopic $m/z$ ratio. This usage includes, in the same ion type, those ions for which the only difference or differences are one or more isotopic substitutions. One of ordinary skill in the mass spectrometry arts will readily know how to recognize isotopic distribution patterns and how to relate or convert such distribution patterns to monoisotopic masses. Occasionally, the word "ion" is used herein in adjective form, as in "precursor-ion mass spectrum" or "product-ion mass spectrum". This latter usage should be understood as referring to any number (one or more) of charged particles- but, generally, a large plurality of such charged particles. Thus, the term "precursor-ion mass spectrum" may be generally understood as referring to a mass spectrum of precursor ions. The term "scan" as used herein is used loosely to refer to any mass spectrum - such as a precursor-ion mass spectrum, a product-ion mass spectrum, both a precursor-ion mass spectrum and an associated product-ion mass spectrum considered together, etc. This terminology usage is employed even though many instances of mass spectrometer instruments that may produce data suitable for analysis according to the present teachings are not, strictly speaking, mass-scanning-type instruments. For instance, the mass spectrometer system **15** illustrated in FIG. 1 is not a mass-scanning type of instrument.

The two elsewhere-disclosed methods, referred to above, are complementary to one another. When the instrument can scan fast enough to sample 7-9 or more points across a chromatographic peak, then lineshape correlation provides excellent results and, in such cases, it is not critical to have ppm accuracy of the mass values. However, when the chromatographic peaks are very narrow with respect to the sampling rate, but the instrument is capable of high mass accuracy or precision, then the neutral loss correlation method works well. The reconstructed MS-2 spectra obtained by this procedure of choosing between either lineshape correlation or neutral-loss correlation are very high quality since the correlation analysis removes chemical noise and produces "clean" MS-2 spectra which may be easily assigned to actual structures, and which, more importantly, are very reproducible.

The reproducibility of the decomposed reconstructed MS-2 spectra generated according to the present teachings enables recognition of spectra corresponding both to previously-analyzed compounds as well as recognition of never-before-observed compounds. Decomposed reconstructed MS-2 spectra may be written to a database when there is at least one product-ion mass in the MS-2 spectrum. In a sample of 10 typical raw files (average size 57 MB) generated by an Exactive™ mass spectrometer, 2785 such spectra were found, or on average, about 280 spectra per data file. This value of 280 spectra/file corresponds to a data compression from 570 MB to approximately 300 KB ( a compression of more than 1000:1), so months worth of data can be stored, but more importantly, this data can easily and quickly be searched for a match. Other types of data may be more component-rich; some other data files have been examined that yield five times the number of components as the data mentioned above (when measured as the number of valid MS-2 spectra per MB of file size), but that still produces a compression of almost 500:1.

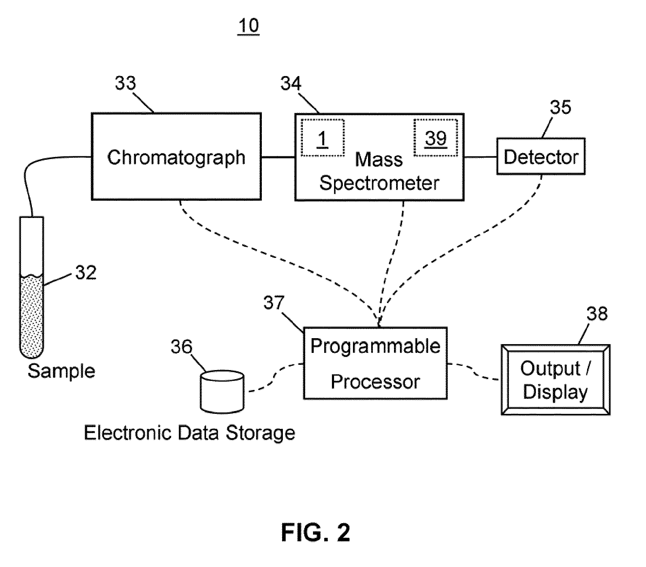

The automated methods and apparatus described herein do not require any user input or intervention. The following description is presented to enable any person skilled in the art to make and use the invention, and is provided in the context of a particular application and its requirements. Various modifications to the described embodiments will be readily apparent to those skilled in the art and the generic principles herein may be applied to other embodiments. Thus, the present invention is not intended to be limited to the embodiments and examples shown but is to be accorded the widest possible scope in accordance with the features and principles shown and described. The particular features and advantages of the invention will become more apparent with reference to the appended FIGS. 2-12, taken in conjunction with the following description.

General Considerations

FIG. 2 is a schematic diagram of a system for generating and automatically analyzing chromatography / mass spectrometry spectra in accordance with the present teachings. A chromatograph **33,** such as a liquid chromatograph, high-performance liquid chromatograph or ultra high performance liquid chromatograph receives a sample **32** of an analyte mixture and at least partially separates the analyte mixture into individual chemical components, in accordance with well-known chromatographic principles. As a result, the at least partially separated chemical components are transferred to a mass spectrometer **34** at different respective times for mass analysis. As each chemical component is received by the mass spectrometer, it is ionized by an ionization source of the mass spectrometer. The ionization source may produce a plurality of ions (i.e., a plurality of precursor ions) comprising differing charges or masses from each chemical component. Thus, a plurality of ions of differing mass-to-charge ratios may be produced for each chemical component, each such component eluting from the chromatograph at its own characteristic time. These various ions are analyzed and detected by the mass spectrometer together with its detector **35** and, as a result, appropriately identified according to their various mass-to-charge ratios. As illustrated in FIG. 1, the mass spectrometer comprises a reaction cell to fragment or cause other reactions of the precursor ions but may lack a mass filtering step for selection of particular ions to introduce into the reaction cell. In such a situation, the reaction cell, instead, causes reactions to or fragmentation of all ions at once.

Still referring to FIG. 2, a programmable processor **37** is electronically coupled to the detector of the mass spectrometer and receives the data produced by the detector during chromatographic / mass spectrometric analysis of the sample(s). The programmable processor may comprise a separate stand-alone computer or may simply comprise a circuit board or any other programmable logic device operated by either firmware or software. Optionally, the programmable processor may also be electronically coupled to the chromatograph and/or the mass spectrometer in order to transmit electronic control signals to one or the other of these instruments so as to control their operation. The nature of such control signals may possibly be determined in response to the data transmitted from the detector to the programmable processor or to the analysis of that data. The programmable processor may also be electronically coupled to a display or other output **38,** for direct output of data or data analysis results to a user, or to electronic data storage **36.** The programmable processor shown in FIG. 2 is generally operable to: receive a precursor ion chromatography / mass spectrometry spectrum and a product ion chromatography / mass spectrometry spectrum from the chromatography / mass spectrometry apparatus and to automatically perform the various data analysis, data retrieval and data storage operations in accordance with the various methods discussed below.

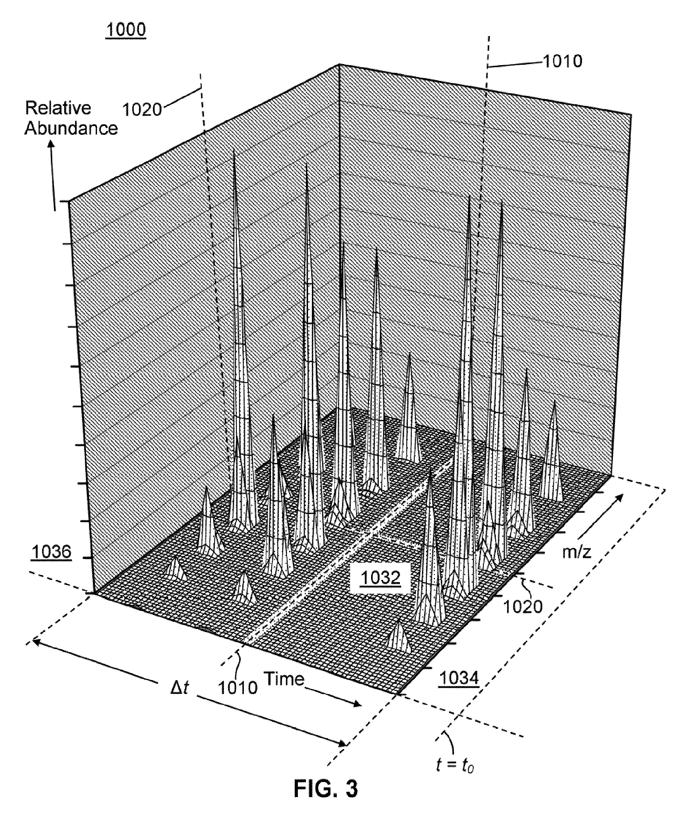

FIG. 3 is a perspective view of a three-dimensional graph of hypothetical LC/MS data. As is common in the representation of such data, the variables time and mass (or mass-to-charge ratio, $m/z$) are depicted on the "floor" of the perspective diagram and the variable representing ion abundance (for instance, detected ion current) is plotted in the "vertical" dimension of the graph. Thus, ion abundance is represented as a function of the other two variables, this function comprising a variably shaped surface above the "floor". Each set of peaks dispersed and in line parallel to the $m/z$ axis represents the various ions produced by the ionization of a single eluting analyte (or, possibly, of fortuitously co-eluting analytes) at a restricted range of time. In a well-designed chromatographic experiment, each analyte of a mixture will elute from the column (thereby to be mass analyzed) within a particular diagnostic time range. Consequently, either a single peak or a line of mass-separated peaks, each such peak representing a particular ion produced by the eluting analyte, is expected at each elution time (or retention time) range.

For clarity, only a very small number of peaks are illustrated in FIG. 3. In practice, data obtained by a chromatography-mass spectrometry experiment may comprise a very large volume of data. A mass spectrometer may generate a complete "scan" over an entire mass range of interest in a matter of tens to hundreds of milliseconds. As a result, up to several hundred complete mass spectra may be generated every second. Further, the various analytes may elute over a time range of several minutes to several tens of minutes, depending on the complexity of the mixture under analysis and the range of retention times represented.

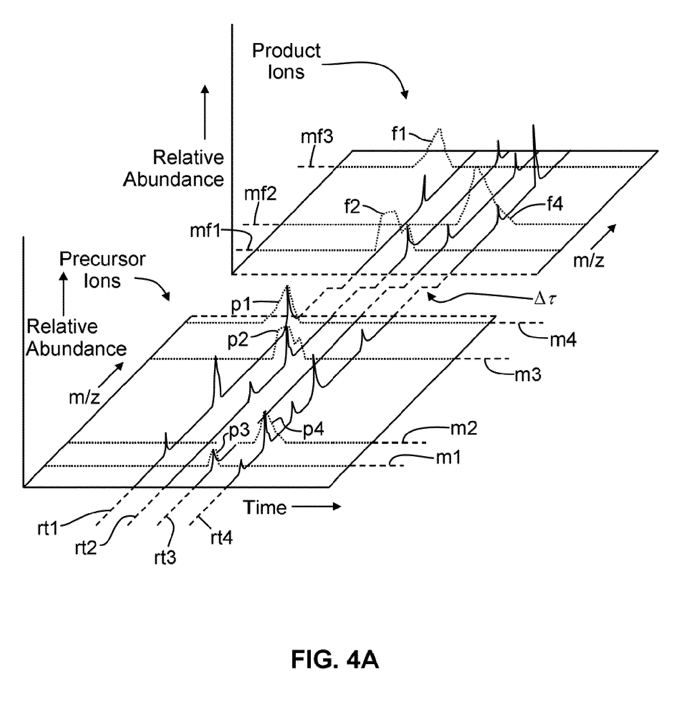

When the chromatography-mass spectrometry experiment and data generation are performed by a mass spectrometer system that performs both all-ion precursor ion scanning and all-ions product ion scanning, the data for each eluate will logically comprise two data subsets which are interleaved with one another in time, each of which is similar to the data set illustrated in FIG. 3. One of these data subsets will contain the data for the precursor ions and the other data subset will contain the data for the product ions. Such a situation is illustrated schematically in FIGS. 4A-4C, discussed in greater detail in following paragraphs. Generally, the data set containing the product ion peaks will also contain some peaks corresponding to residual un-fragmented or un-reacted precursor ions.

Returning to the discussion of FIG. 3, the data depicted therein may comprise an entire stored data file representing results of a prior experiment. Alternatively, the data represent a portion of a larger data set in the process of being acquired by an LC/MS instrument. For instance, the data depicted in FIG. 3 may comprise recently collected data held in temporary computer readable memory, such as a memory buffer, and corresponding to an analysis time window, $\Delta t$, upon which calculations are being formed while, at the same time, newer data is being collected. Such newer, not-yet-analyzed data is represented, in time and $m/z$ space, by region **1034** and the data actually being collected is represented by the line $t=t_0$. Older data which has already been analyzed by methods of the present teachings and which has possibly been stored to a permanent computer readable medium, is represented by region **1036.** With such manner of operation, methods in accordance with the present teachings are carried out in near-real-time on an apparatus used to collect the data or using a processor (such as a computer processor) closely linked to the apparatus used to collect the data.

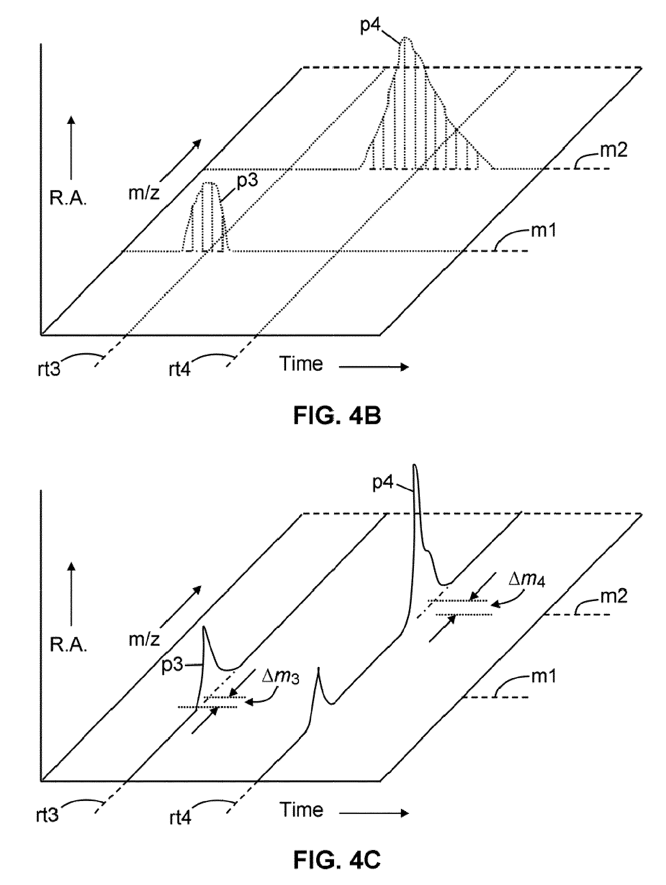

Operationally, data such as that illustrated in FIG. 3 is collected as separate mass spectra (also referred to herein as "scans"), each mass spectrum (scan) corresponding to a particular respective time point. Such mass spectra may be envisioned as residing within planes parallel to the plane indicated by the trace lines **1010** in FIG. 3 or parallel to the lines **rt1, rt2, rt3** and **rt4** in FIG. 4A. As illustrated in FIG. 4A, each precursor-ion scan corresponds to a respective product-ion scan. Once at least a portion of data has been collected, such as the data in region **1032** in FIG. 3, then the information in the data portion may be logically re-organized as extracted ion chromatograms (or, at least portions thereof). Each such XIC may be envisioned as a cross section through the data in a plane parallel to the plane indicated by trace lines **1020** in FIG. 3 or parallel to the lines **m1, m2, m3, m4, mf1, mf2,** and **mf3** in FIG. 4A. Hypothetical extracted ion chromatograms are shown as dotted lines in FIG. 4A and FIG. 4B. Each XIC represents the elution profile, in time, of ions of a particular mass-to-charge range. The XIC representation of the data is useful for understanding the methods of the present teachings.

Several schematic hypothetical XIC profiles are shown in FIGS. 4A-4C. These profiles include several example peaks. The illustrated precursor scan peaks are peak **p1** at coordinates (rt1, m4), peak **p2** at coordinates (rt2, m3), peak **p3** at coordinates (rt3, m1) and peak **p4** at coordinates (rt4, m2). Three product ion scan peaks are also illustrated: peak **f1** at coordinates (rt1, mf3), peak **f2** at coordinates (rt2, mf1) and peak **f4** at coordinates (rt4, mf2). As described above with respect to the operation of the spectrometer system **15** (FIG. 1), the precursor-ion and product-ion scans alternate in time. Thus, even though the time lines **rt1, rt2, rt3** and **rt4** correspond to the maximum production of precursor ions of different nearly-co-eluting compounds, the respective immediately following product ion scans are offset in time, relative to the maxima, by a time delay increment $\Delta\tau$. The system **15** illustrated in FIG. 1 is capable of repeating the precursor scan and product ion scan sequence five times for compounds that elute over a period of 1 second (that is, 10 total scans per second). Thus, even though precursor ion and product ion scans are not coincident in time, there are generally a sufficient number of precursor ion scans and product ion scans to permit discernment of the profiles of the peaks.

General Procedure

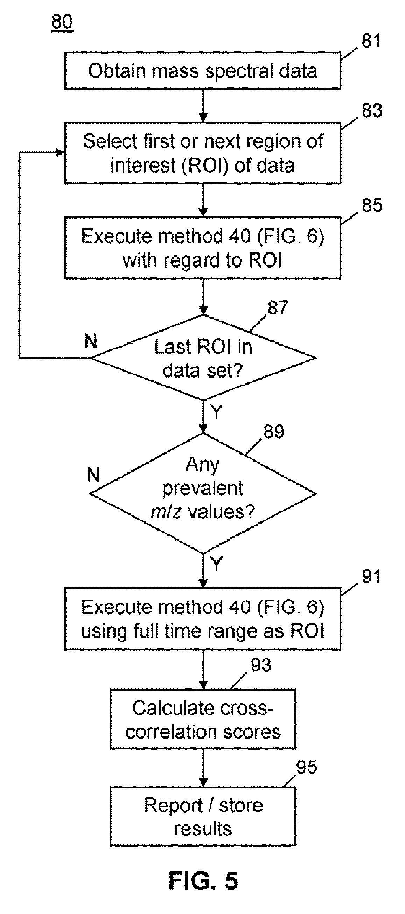

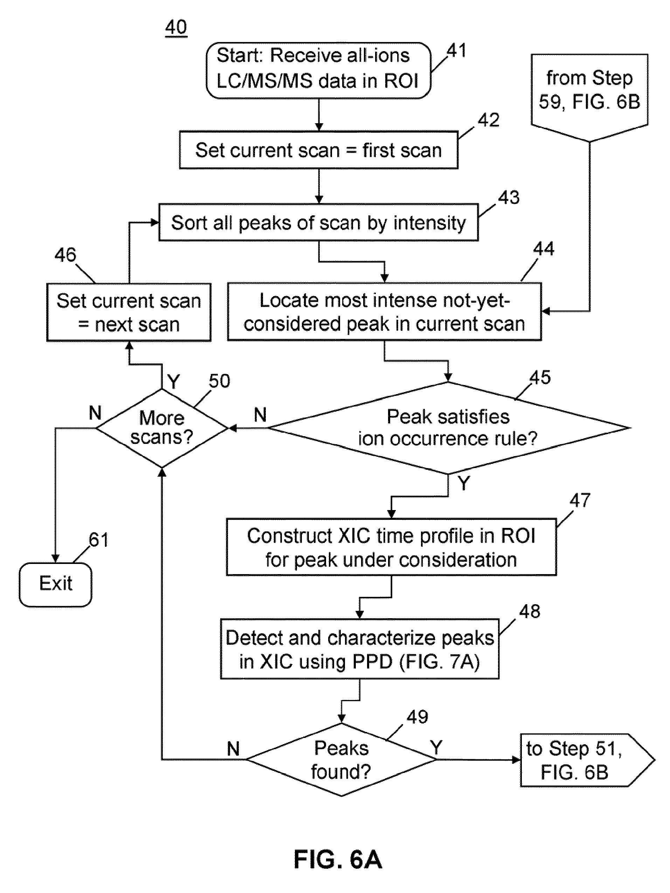

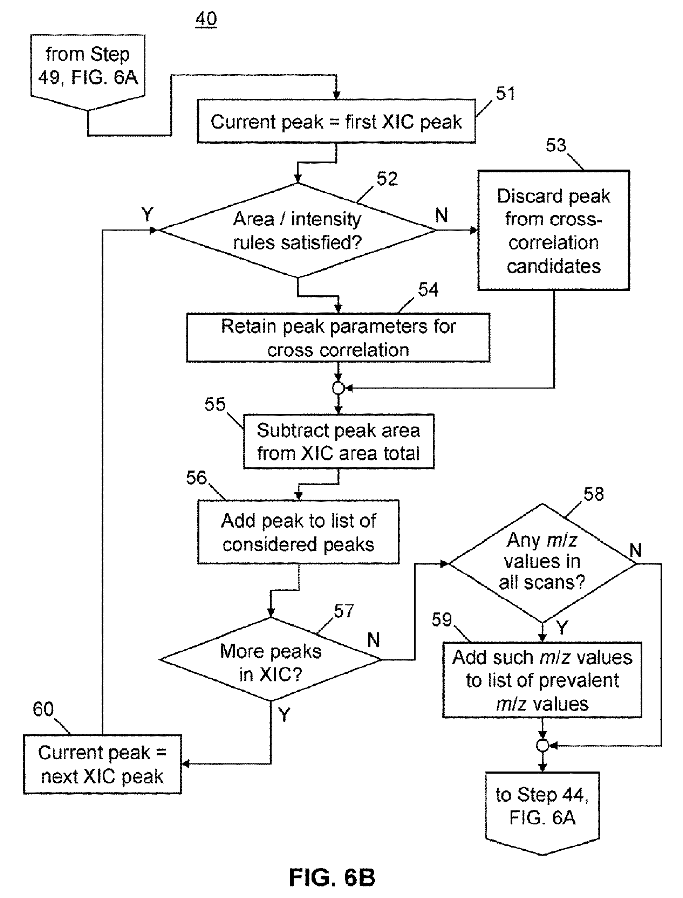

FIG. 5 is a flow chart of a first general method **80** for handling and analyzing mass spectral data in accordance with the present teachings. In Step **81,** of the method **80,** mass spectral data is obtained, either by acquiring new data directly from a mass spectrometer during the course of an experiment or, alternatively, by inputting previously-generated or previously-observed data from a data file or from a data storage device. In Step **83,** a first region of interest (ROI) is selected, the region of interest including data within a particular time slice or time window. For example, the region **1032** illustrated in FIG. 3 may comprise such a region of interest. In Step **85,** the data within the currently selected ROI is analyzed in accordance with the method **40** illustrated in FIG. 6 and discussed below in reference to that figure. In brief, the method **40** is a method for generating automated correlations between all-ions precursor ions and all-ions-fragmentation product ions within the selected region of interest. After the determination of any and all such correlations within the currently selected ROI in Step **85,** if there are additional regions of interest to be considered (Step **87),** execution returns to Step **83** and the next ROI is selected in considered in turn.

After all regions of interest have been considered, then execution of the method **80** proceeds to Step **89** in which the existence of any potential "prevalent *m/z* values" is noted. As used herein, the term "prevalent *m/z* value" refers to any *m/z* value that is associated with a mass chromatogram peak that either is too broad in time to be fully encompassed by any of the regions of interest analyzed in Step **85.** Since the edges of such a peak will not both be observed in any one region of interest, correct characterization of such a peak is not possible when employing the peak detection routines of the method **40** (discussed further below) in conjunction with data in a single ROI. Although such peaks cannot be properly characterized in any one ROI, their existence may nonetheless be noted (and recorded) by the prevalence of above-baseline signal in association with one or more particular *m/z* values within all mass scans within a region of interest (see Steps **58** and **59** of the method **40** discussed in greater detail below). Accordingly, in Step **91** of the method **80,** the method **40** (FIG. 6) is executed once again whereby, in this case, the entire time range of the mass spectral data is considered to comprise a new region of interest. Execution of the method **40** in Step **91** in this fashion permits proper characterization of those mass chromatogram peaks which do not fully reside within any one region of interest selected in Step **83.**

After execution of the Steps **81-91** of the method **80** (FIG. 5), parameters of synthetic fit peaks to certain of the chromatogram peaks within the data set will be available for further analysis. Specifically, these fit parameters will be available for those chromatogram peaks which satisfy certain criteria, as discussed in greater detail in the discussion below relating to the method **40.** The parameters of synthetic fit peaks are stored in Step **54** of the method **40** (see FIG. 6B) as suitable peaks in extracted ion chromatograms are identified. These parameters are then used to calculate cross-correlation scores in Step **93** of the method **80** (see FIG. 5) and these correlation scores are used to identify correlated precursor and product ions. In brief, the cross correlation for each retained peak is calculated with respect to every other mass that formed an XIC peak. Each detected precursor peak is considered, through a cross-correlation calculation, against each detected product ion peak in order to match precursor ions with product ions. The details of the calculations are presented in a subsequent section herein.

Finally, the results of the calculations or identifications are then reported or stored in Step **95.** The results may include calculated product/precursor matches, information regarding detected peaks or other information. The reporting may be performed in numerous alternative ways-for instance via a visual display terminal, a paper printout, or, indirectly, by outputting the parameter information to a database on a storage medium for later retrieval by a user. The reporting step may include reporting either textual or graphical information, or both. Reported peak parameters may be either those parameters calculated during the peak detection step or quantities calculated from those parameters and may include, for each of one or more peaks, location of peak centroid, location of point of maximum intensity, peak half-width, peak skew, peak maximum intensity, area under the peak, etc. Other parameters related to signal to noise ratio, statistical confidence in the results, goodness of fit, etc. may also be reported in Step **61.** The information reported in Step **95** may also include characterizing information on one or more analytes and may be derived by comparing the results obtained by the methods described herein to known databases. Such information may include chemical identification of one or more analytes (e.g., ions, molecules or chemical compounds), purity of analytes, identification of contaminating compounds, ions or molecules or, even, a simple notification that an analyte is (or is not) present in a sample at detectable levels.

Lineshape Correlation Methods

As briefly noted in the previous paragraphs, FIGS. 6A-6B present a flowchart of a first method **40** for generating automated correlations between all-ions precursor ions and all-ions-fragmentation product ions in accordance with the present teachings. In the initial step, Step **41** (FIG. 6A), all-ions LC/MS/MS data generated by a chromatograph-mass spectrometer apparatus is received (for example, from either Step **85** or Step **91** of the method **80** shown in FIG. 5). The LC/MS data comprises two data subsets, as shown in FIG. 4A - one data subset containing data for precursor ions and the other data subset containing data for all the fragment ions formed by reaction or fragmentation of all the precursor ions. Each data subset comprises ion abundance (or relative abundance) information as a function of time and *m/z*.

The calculations of method **40** are performed on a chosen time window of the data set. This time-window corresponds to a current region of interest (ROI) of recently collected data, such as region **1032** of FIG. 3. The region of interest includes data (e.g., see FIG. 4A) from the precursor ion scan (MS scan) as well as the fragment ion scan (MS/MS scan). In embodiments, this window is 0.6 minutes wide. This time windows represent a small portion of a typical chromatographic experiment which may run for several tens of minutes to on the order of an hour. In some implementations, data dependent instrument control functions may be performed in automated fashion, wherein the results obtained by the methods herein are used to automatically control operation of the instrument at a subsequent time during the same experiment from which the data were collected. For instance, based on the results of the algorithms, a voltage may be automatically adjusted in an ion source or a collision energy (that is applied to ions in order to cause fragmentation) may be adjusted with regard to collision cell operation. Such automatic instrument adjustments may be performed, for instance, so as to optimize the type or number of ions or ion fragments produced.

The data of the region of interest may be systematically examined in the time window, by searching for peaks to be tested by subsequent cross-correlation calculation. For example, an algorithm in accordance with the present teachings may progress through the data, scan-by-scan, and in two parallel processes, one for each scan type (i.e., precursor ions and fragment ions). In the present example, the window width is only 0.3 minutes wide at time zero since there is no data before time=0. As scans of higher time are examined, the window increases until the scan at time 0.3 minutes uses a window of the specified 0.6 minutes. In practice the time window width may vary widely.

In Step **42** of the exemplary method **40** (FIG. 6A), the scan to be examined (the current scan) is set to be the initial scan within the ROI. This is an initialization step for a loop in which scans are sequentially examined. In Step **43,** the peaks of the current scan are sorted by intensity and the ions are examined one by one, starting with the most intense (Step **44).** In general, all ions are examined, but for very rapid work or strong signals, a threshold may be applied and only ions with intensities above threshold examined. In the present example, Step **59** (described in greater detail later in this document) is performed when all ions in all scans of the ROI have been examined. In Step **45,** the occurrence of an ion is noted, and its history or time-profile is compared to a rule for ions to be considered as forming a peak. A preferred rule that is used is that the ion must occur in three contiguous scans (scans of the same type), but any rule based on ion appearance and scan number may be used. For example, a rule that the ion must appear in 3 of 5 contiguous scans might alternatively be chosen. (Ions are considered identical if they agree within the mass tolerance, and as an ion history is accumulated, any new occurrence is compared to the average value of the previous instances, not simply the previous instance.)

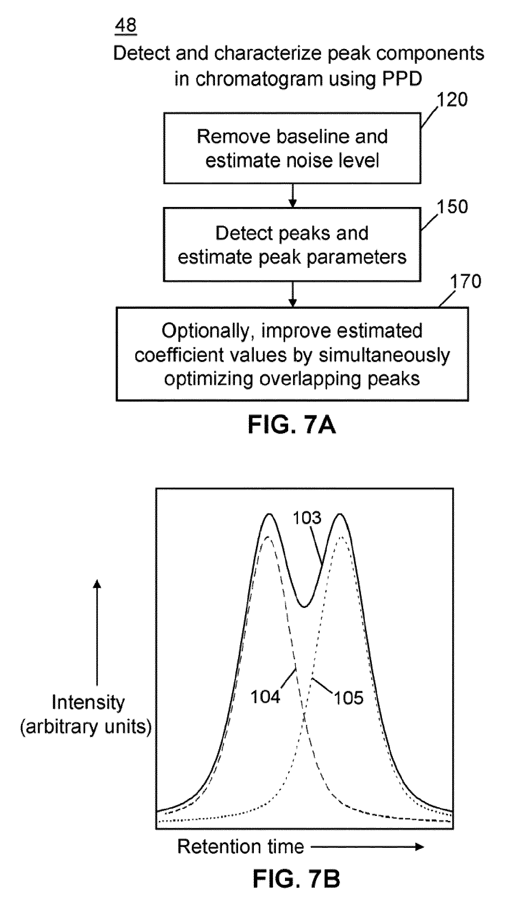

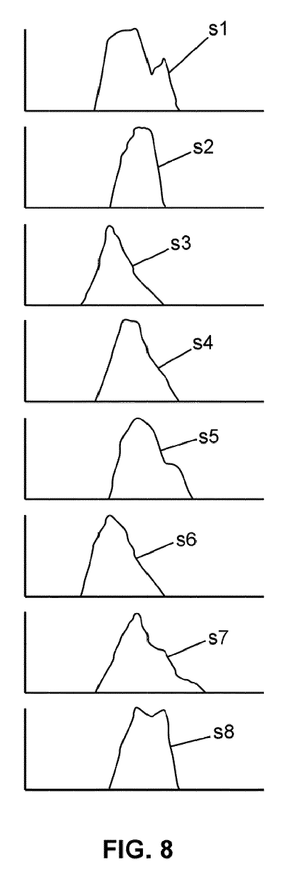

If, in Step **45,** the peak does not satisfy the ion occurrence rule, then, if there are more unexamined scans in the ROI (determined in Step **50),** the current scan is set to be the next unexamined scan (Step **46)** and the method returns to Step **43** to begin examining the new current scan. If the ion occurrence rule (as determined in Step **45)** is satisfied, then an extracted ion chromatogram (XIC) corresponding to the mass range of the ion peak under consideration is constructed in Step **47.** It is to be noted that the terms "mass" and "mass-to-charge" ratio, as used here, actually represent a small finite range of mass-to-charge ratios. The width or "window" of the mass-to-charge range is the stated precision of the mass spectrometer instrument. The technique of Parameterless Peak Detection (PPD, see FIG. 7A and additional discussion in U.S. Patent No. 7,983,852 assigned to the assignee of the instant invention and incorporated herein in its entirety) may then be employed to find peaks in an extracted ion chromatogram (XIC) corresponding to this time window in Step **48.** Once this particular mass has been tested for peaks in the XIC, it is not tested again until the center of the time window has increased by the window size. (So, for example, if an ion is tested for peaks when the time window is 2-2.6, it will not be tested again until the window is 2.6-3.2.) The dectecting and characterizing step (Step **48)** may employ, without limitation, Parameterless Peak Detection as described in U.S. Patent No. 7,983,852 in order to decompose a chromatogram trace comprising overlapping or partially overlapping peaks within the XIC under consideration. This decomposition enables separating of the effects of co-eluting compounds (see FIG. 8 and the discussion relating thereto).

If, in the decision step, Step **49,** no component peaks are found by PPD for the mass under consideration, then, if there are remaining unexamined scans (Step **50),** the method returns back to Step **46** and then Step **43.** However, if peaks are found, then the method continues to Step **51** (FIG. 6B) in which the first of possibly several peaks in the XIC is set for initial consideration. In the next step, Step **52,** for each peak found by PPD, additional rules of large relative area and high relative intensity (described in further detail in the next paragraph) are applied. Peaks that fail these tests are discarded (Step **53),** whereas those that pass are accepted and have their descriptive parameters retained (Step **54)** for further processing by cross-correlation score calculations (such as in Step **93** of method **80** shown in FIG. 5). Regardless of whether or not a peak is accepted, after each peak is considered, the peak area of the peak is subtracted (Step **55)** from the total area used in the relative area criterion in subsequent iterations of Step **52.** Also (Step **56)** the peak is added to a list of peaks within the ROI that have been examined, to prevent possible duplicate consideration of a single peak.

The Step **52** of the method **40** is now discussed in more detail. In Step **52,** the area of, $A_j$, of the peak currently under consideration (the $j^{th}$ peak) is noted. Also, the total area ($\Sigma A$) under the curve the fitted extracted-ion chromatogram and the average peak signal intensity ($I_{ave}$) at the locations of any remaining peaks in the fitted chromatogram are calculated. The area $\Sigma A$ is the area of the data remaining after any previously considered peaks have been detected and removed. The Step **52** compares the area, $A_j$, of the most recently found peak to the total area ($\Sigma A$). Also, this step compares the peak maximum intensity, $I_j$, of the most recently found peak is compared to $I_{ave}$. If it is found either that $(A_j / \Sigma A) < \omega$ or that $(I_j / I_{ave}) < \rho$, where $\omega$ and $\rho$ are pre-determined constants, then the execution of the method **40** branches to Step **53** in which the peak is removed from a list of peaks to be considered in - and is thus eliminated from consideration in - the subsequent cross-correlation score calculation step. The removal of certain peaks in this fashion renders the fitted peak set consistent with the expectations that, within an XIC, each actual peak of interest should comprise a significant peak area, relative to the total peak area and should comprise a vertex intensity that is significantly greater than the local average intensity.

Returning to the discussion of the method **40** (FIG. 6B), it may be noted that if more peak components exist in the XIC under consideration (decision Step **57),** then the method branches to Step **60** in which the next XIC peak component is set for consideration and then back to Step **52.** If, however, no additional peaks remain the XIC, then execution proceeds to Step **58,** in which a determination is made regarding whether or not any $m/z$ values are associated with significant signal intensity above baseline in all scans (i.e., mass spectra) of the current XIC within the current ROI. As discussed in a previous section, such $m/z$ values will likely correspond to "prevalent $m/z$ values" that are associated with chromatogram peaks spanning a range of time greater than the range of time of the current ROI. Any such possible prevalent $m/z$ values are noted in Step **59.** After execution of either Step **58** or Step **59,** execution returns to Step **44** (FIG. 6A) so as to continue examining additional peaks (if any) in the current ROI. The above-described sequence continues until all peaks in the current ROI have been examined and, consequently, all precursor ion peaks or product ion peaks to be used for matching have been identified. In this exemplary method, if no further scans exist within the region of interest (Step **50),** then the method terminates at Step **61.**

The method **40** diagrammed in FIGS. 6A-6B provides a high-level overview of generating automated correlations between all-ions precursor ions and all-ions-fragmentation product ions. At a lower level, the Step **48** includes detecting and locating peaks in various extracted-ion-chromatogram (XIC) representations of the precursor ion and product ion data and may itself be regarded as a particular method, which is shown in flowchart form in FIG. 7A. Since each XIC includes only the single independent variable of time (e.g., Retention Time), this section is thus directed to detection of peaks in data that includes only one independent variable. The various sub-procedures or sub-methods in the method **48** may be grouped into three basic stages of data processing, each stage possibly comprising several steps as illustrated in FIG. 7A. The first step, Step **120,** of the method **48** is a preprocessing stage in which baseline features may be removed from the received chromatogram and in which a level of random "noise" of the chromatogram may be estimated. The next Step **150** is the generation of an initial estimate of the parameters of synthetic peaks, each of which models a positive spectral feature of the baseline corrected chromatogram. Such parameters may relate, for instance, to peak center, width, skew and area of modeled peaks, either in preliminary or intermediate form. The subsequent optional Step **170** includes refinement of fit parameters of synthetic peaks determined in the preceding Step **150** in order to improve the fit of the peaks, taken as a set, to the baseline corrected chromatogram. The need for such refinement may depend on the degree of complexity or accuracy employed in the execution of modeling in Step **150.** The optional refinement step comprises exploring the space of all parameters across all peaks to find the set of values that minimizes the sum of squared differences between the observed and model chromatogram. This contrasts somewhat with the procedure employed in Step **150,** in which peaks are detected and modeled individually or in pairs. Preferably, the squared difference may be calculated with respect to the portion of the chromatogram comprising multiple or overlapped peaks. It may also be calculated with respect to the entire chromatogram. The model chromatogram is calculated by summing the contribution of all peaks estimated in the previous stage.

The purpose of the method **48,** as outlined in FIG. 7A, is to decompose a chromatogram trace into component peaks, such as the peaks **104** and **105** schematically illustrated in FIG. 7B. The Step **48** is outlined in brief in FIG. 7A. The individual steps shown in FIG. 7A are discussed in much greater detail in the aforementioned United States Patent 7,983,852.

Several schematic extracted ion chromatograms are illustrated in FIG. 4A by dotted lines residing at respective mass-to-charge values indicated by sections **m1, m2, m3** and **m4** as well as at mass-to-charge values indicated by sections **mf1, mf2** and **mf3.** Subsequent to execution of the methods discussed above, each such XIC is defined by the set of synthetic peaks calculated by those methods. The hypothetical synthetic extracted ion chromatograms schematically shown in FIG. 4A illustrate elution of various ionized chemical constituents at closely-spaced times **rt1, rt2, rt3** and **rt4.** Although illustrated as separated times, one or more of the times **rt1, rt2, rt3** and **rt4** could even be identical to one another, such that the various chemical constituents are co-eluting constituents. It should be noted that the mass scale (i.e., *m/z* scale) relating to product ion scans in FIG. 4A is not a simple extension of the mass scale relating respectively relating to precursor ion scans. In fact, the two mass scales may overlap one another but are not necessarily identical to one another.

The extracted ion chromatogram (XIC) peak shapes for components that elute at similar times are neither all the same, nor are they all different. FIG. 8 shows results from a typical situation, in which the peak shapes in various extracted ion chromatograms fall into several groups of patterns indicated by the peak profiles **s1-s8.** The data from which these profiles were generated was obtained using an instrumental system similar to that shown in FIG. 2 and were obtained during the 6-second elution of a single mass chromatographic peak of a 500 nM solution of the drug Buspirone. The profiles **s1-s8** correspond to different respective *m/z* ranges obtained from the all-ions fragment data.

Comparison of the illustrated XIC peak profiles in FIG. 4A illustrates how precursor-ion profiles may be similar in shape to the profiles of product ions relating to elution of the same compound and, also, how profiles relating to elution of different compounds may be expected to have different respective shapes. Since the chemistry and physics that determine the chromatographic peak shape are unique for each molecule and cease when the molecule exits the column, one can expect that XICs having similar shapes may be related. A stronger statement can be made that XICs that have different shapes are not fragments of the same precursor. By using Parameterless Peak Detection (PPD) techniques, as described in Section 2 herein, to characterize the peak shape, small differences in shape can be encoded in a correlation vector (described in more detail following). This can be enhanced by additional smoothing after the peak is detected (but not before, since prior smoothing can smooth a noise spike into a peak). Step **93** of method **80** (FIG. 5) is the cross-correlation step which is described in more detail in the following paragraphs.

Overall cross-correlation scores (CCS) in accordance with the present teachings are calculated (i.e., in Step **93** of method **80)** according to the following strategy. For each mass in the experimental data that is found to form a chromatographic peak by PPD as described in Section 2, the cross correlation of every mass with every other mass is computed. In the present context, the term "peak" refers simply to masses that have non-zero intensity values for several contiguous or nearly contiguous scans (for example, the scans at times **rt1, rt2, rt3** and **rt4** illustrated in FIG. 4A) of the same filter type. Each cross-correlation score may include a peak shape correlation score (calculated in terms of a time-versus-intensity for each mass that forms a recognized peak), a mass defect correlation score (for differences along the *m/z* axis) and an optional peak width correlation score as described below. The final correlation score may be calculated as a weighted average of the peak shape correlation, mass defect correlation and peak width correlation scores. If a calculated overall correlation score is such that a match between masses is recognized, then an MS/MS spectrum is reconstructed with the mass of the precursor-ion member as parent and masses from the all-ions fragmentation step as product ions.

The calculation of peak-shape cross correlations may use a trailing retention time window. The calculation makes use of a numerical array including mass, intensity, and scan number values for every mass that forms a chromatographic peak. As described previously in this document, Parameterless Peak Detection (PPD) is used to calculate a peak shape for each mass component. This shape may be a simple Gaussian or Gamma function peak, or it may be a sum of many Gaussian or Gamma function shapes, the details of which are stored in a peak parameter list. Once the component peak shape has been characterized by an analytical function (which may be a sum of simple functions), the problem of calculating a dot-product correlation is greatly simplified. Time offsets (e.g., $\Delta\tau$, see FIG. 4A) in the original data are no longer relevant, since the analytic functions for two different mass components may be sampled at the same arbitrary time points. Once this is accomplished, it is trivial to calculate a cross correlation, here considered as a simple vector product ("dot product"). These cross correlations are normalized by also calculating, and dividing by, the autocorrelation values. Consequently, the peak shape correlation (PSC) between two peak profiles, p1 and p2 (denoted, functionally as $p1(t)$ and $p2(t)$, where $t$ represents a time variable, may be calculated as

$$\text{PSC}(p1, p2) = \frac{\sum\limits_{j=j\min}^{j=j\max}\left[p1(t_j) \times p2(t_j)\right]}{\left\{\sum\limits_{j=j\min}^{j=j\max} p1(t_j)^2\right\}^{1/2}\left\{\sum\limits_{j=j\min}^{j=j\max} p2(t_j)^2\right\}^{1/2}} \qquad \text{Eq. 1}$$

in which the time axis is considered as divided into equal width segments, thus defining indexed time points, $t_j$, ranging from a practically defined lower time bound, $t_{j\min}$, to a practically defined upper time bound, $t_{j\max}$. Accordingly, the quantity PSC can theoretically have a range of 1 (perfect correlation) to -1 (perfect anti-correlation), but since negative going chromatographic peaks are not detected by PPD (by design) the lower limit is effectively zero. For example, the lower and upper time bounds, $t_{j\min}$, and, $t_{j\max}$, may be set in relation to each precursor ion. In such a case, the time values are chosen so as to sample intensities a fixed number of times (for instance, between roughly seven and fifteen times, such as eleven times) across the width of a precursor ion peak. The masses to be correlated with the chosen precursor ion then use the same time points. This means that if these masses form a peak at markedly different times, the intensities will be essentially zero. Partially overlapped peaks will have some zero terms.

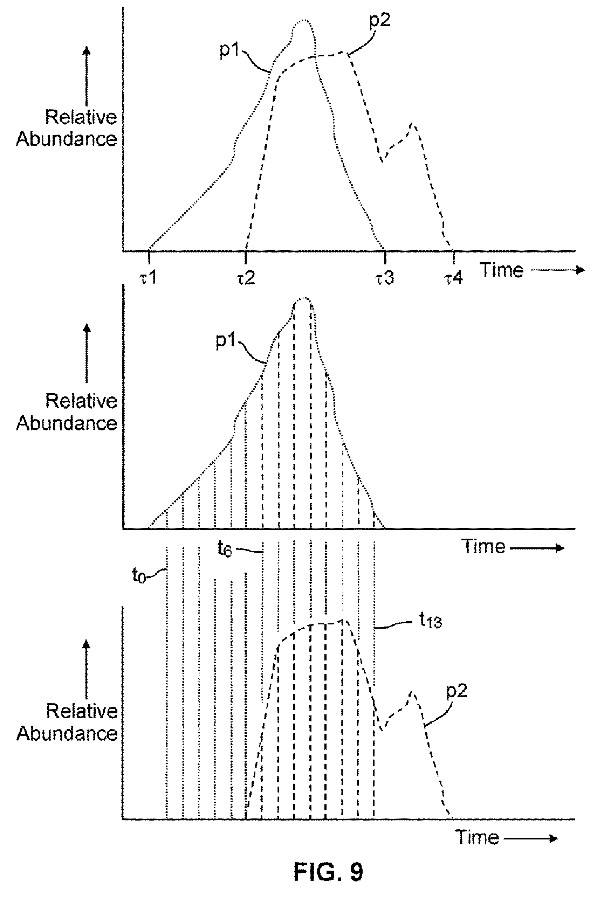

FIG. 9 graphically illustrates calculation of a dot product cross-correlation score in this fashion. In FIG. 9, two XIC peak profiles **p1** and **p2** are reproduced from FIG. 4. Peak **p1** has appreciable intensity above baseline only between time points $\tau1$ and $\tau3$ and peak **p2** has appreciable intensity only between time points $\tau2$ and $\tau4$. Assume that peak profile **p1** corresponds to a precursor ion (or precursor ion candidate) and that peak **p2** corresponds to a product ion (or product ion candidate). As discussed above, to calculate the dot-product cross correlation score between these two peaks, the retention time axis may be considered as being divided into several equal segments between time points $\tau1$ and $\tau3$, thereby defining, in this example, indexed time points $t_j$ where ($0 \le j \le 13$). The two peak profiles are shown separately in the lowermost two graphs of FIG. 9 in association with vertical lines representing the various indexed time points along the retention time axis. In this representation, peak **p2** only has appreciable intensity between the points $t_6$ and $t_{(13)}$. Thus, in this example, the peak shape correlation is given by

$$\text{PSC}(p1, p2) = \frac{\sum\limits_{j=0}^{j=13}\left[p1(t_j) \times p2(t_j)\right]}{\left\{\sum\limits_{j=0}^{j=13} p1(t_j)^2\right\}^{1/2}\left\{\sum\limits_{j=0}^{j=13} p2(t_j)^2\right\}^{1/2}}$$

Under such a calculation, the cross-correlation score, as calculated above, for the peaks **p1** and **p2** illustrated in FIG. 9 would be a positive number because the peaks partially overlap, but would be below a threshold score for recognizing a peak match, since the peaks have different shapes. The cross-correlation score for a peak with itself or with a scaled version of itself is unity. Note from FIG. 4A that, by this measure, the peaks **p4** and **f4** would have a high cross-correlation score even though they have different magnitudes. In the same fashion, peak **p2** would strongly correlate with peak **f2** and peak **p1** would strongly correlate with peak **f1**. By contrast, the cross-correlation score between the peaks **p3** and **p4** illustrated in FIG. 4B would be essentially zero because these peaks have no overlap (every term in the numerator of Eq. 1 would be essentially zero).

The method also may also calculate and include a mass defect correlation. The mass defect is simply the difference, $\Delta m$, between the unit resolution mass and the actual mass, expressed in a relative sense such as parts per million (ppm). Thus the mass defect for a peak, p, can be expressed as:

$$MD_p = 1000000 \times \frac{\Delta m_p}{m_p} \qquad\qquad Eq.\ 2$$

FIG. 4C illustrates how the quantities $\Delta m_3$ and $\Delta m_4$ may be determined for the peaks **p3** and **p4,** respectively. Note that the sign of the mass defect is negative for peak **p3** and positive for peak **p4.** The peaks **p3** and **p4** illustrated in FIG. 4C are the same peaks as illustrated in FIG. 4B, but are shown in profiles parallel to the mass axis instead of the to orthogonal time axis, as in FIG. 4B. Thus, the mass defect provides an independent measure of the potential relatedness of the peaks. This is true in the broadest sense if one considers the mass defect to arise from numerous small contributions from all the atoms in the structure, and the fragments to be of composition typical to the whole. So, for example, an alkane chain that is fragmented will have the same mass defect (on a relative basis) in both halves. On the other hand, chlorobenzene that is fragmented into benzene and chloride ions will have markedly different mass defects.

The mass defect correlation, $MD_{C(p1,p2)}$, between two peaks **p1** and **p2,** is computed simply as

$$MDC_{(p1,p2)} = 1 - A(MD_{p1} - MD_{p2}) \qquad\qquad Eq.\ 3$$

where A is a suitable multiplicative constant. Therefore the mass defect correlation ranges from 1 (exactly the same relative defect) to some small number that depends on the value of *A*.

If it is desired to also use a peak width correlation, which is calculated by a similar formula, using the absolute peak widths as determined by PPD on the XIC peak shapes.

Accordingly, an optional peak width correlation, $PWC_{(p1,p2)}$, between peaks **p1** and **p2** may be calculated by

$$PWC_{(p1,p2)} = 1 - B\,|\,width_{p1} - width_{p2}\,| \qquad\qquad Eq.\ 4$$

in which $B$ is the inverse of the maximum of $width_{p1}$ and $width_{p2}$ and the vertical bars represent the mathematical absolute value operation.

The cross-correlation score, as shown in Step **93** of method **80** (FIG. 5) may be calculated by determining the peak-shape correlation score, PSC, and then optionally combining it together with the mass defect correlation score, MDC, and possibly with the peak width correlation score, PWC, as a weighted average. Accordingly, the overall correlation score, $CCS_{(p1,p2)}$, may be given by

$$CCS_{(p1,p2)} = \{\ X[PSC_{(p1,p2)}] + Y[MDC_{(p1,p2)}] + Z[PWC_{(p1,p2)}]\ \} / \{X + Y + Z\} \qquad Eq.\ 5$$

in which *X, Y* and *Z* are weighting factors. Thus, the overall score, CCS, ranges from 1.0 (perfect match) down to 0.0 (no match). Peak matches are recognized when a correlation exceeds a certain pre-defined threshold value. Experimentally, it is observed that limiting recognized matches to scores to those above 0.90 provides reconstructed MS/MS spectra that match extremely well to experimental spectra.

As one example of how matches recognized from the CSS calculation are used, if a first member of a recognized matched set is a mass from a precursor ion scan, and the list of correlated masses above the 0.90 correlation limit contains 1 additional ion from the precursor ion scan and 4 fragmented ions (in the product ion scan), then 2 potential MS/MS spectra will be reconstructed - one for the first precursor ion mass, and a second for the second precursor ion mass found in the list of correlated masses. For a second example, if the starting mass is found in the product ion scan data and the list of correlated masses contains 4 masses from the precursor ion data and nothing else, then 4 potential MS/MS spectra will be constructed, all having the same product ion but with each having a different precursor mass. It should be pointed out, however, that the actual correlation scores provide a confidence value in the validity of the reconstructed MS/MS spectra, and very often there is a large difference in correlation score between the highest scoring candidate precursor ion and the other candidate precursor ions, making one reconstructed MS2 spectrum easily the most likely correct reconstruction.

It has been found that execution of just the steps described above is very effective and often leads to correct synthetic MS/MS spectra without the necessity of additional analysis. That *m/z* values that are determined gain credibility through their correspondence to plausible chemical formulae. And, since mass spectrometers such as those described herein typically have better precision than accuracy, the criterion used is that the neutral loss mass should correspond to a formula, not the precursor or fragment masses. After mass calibration, of course, all masses should be identified with a formula (or list of formulae), but the calibration step is not necessary when only the neutral loss mass is used.

Since there are typically only 1,000 to 10,000 components in a data file, this calculation is rapid, and the resulting correlation score can be used to eliminate ions that are not closely related to the ion under consideration. Typically only 5-20 masses are highly correlated, and this makes the construction of fragmentation pathways entirely practical.

Neutral-Loss Correlation Methods

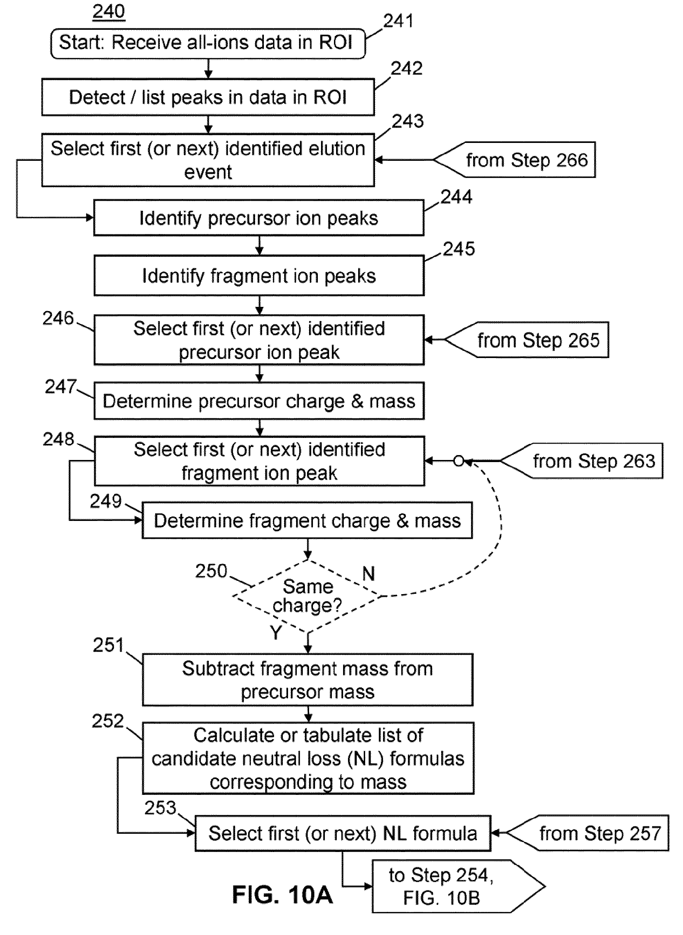

FIGS. 10A-10B present a flowchart of a second method **240** for generating automated correlations between all-ions precursor ions and all-ions-fragmentation product ions in accordance with the present teachings. In the initial step, Step **241** (FIG. 10A), all-ions LC/MS/MS data is generated by and received from a chromatograph-mass spectrometer apparatus. The calculations of method **240** are performed on a chosen time window of the data set. This time-window corresponds to a current region of interest (ROI) of recently collected data, such as region **1032** of FIG. 3. The region of interest includes data from the precursor ion scan (MS scan) as well as the fragment ion scan (MS/MS scan). Thus, the LC/MS data may comprise two data subsets - one data subset containing data for precursor ions and the other data subset containing data for all the fragment ions formed by reaction or fragmentation of all the precursor ions. Each data subset comprises ion abundance (or relative abundance) information as a function of time and $m/z$.

The system **15** illustrated in FIG. 1 is capable of repeating the precursor scan and product ion scan sequence five or more times for compounds that elute over a period of 1 second (that is, 10 or more total scans per second). Thus, even though precursor ion and fragment or product ion scans are not exactly coincident in time, the time offset ($\Delta\tau$ in FIG. 4A) between the acquisition of the precursor ion data and the subsequent product ion data may be considered to be, for purposes of this disclosure, sufficiently small so as to be inconsequential. In those cases in which the chromatic separation and resolution is sufficiently good that the time offset between acquisition of precursor and fragment or product ion data may, in fact, be of consequence, then the XIC correlation methods discussed above may be used to advantage.

In embodiments, the time window corresponding to each ROI is 0.6 minutes wide. This time windows represent a small portion of a typical chromatographic experiment which may run for several tens of minutes to on the order of an hour. In some implementations, data dependent instrument control functions may be performed in automated fashion, wherein the results obtained by the methods herein are used to automatically control operation of the instrument at a subsequent time during the same experiment from which the data were collected. For instance, based on the results of the algorithms, a voltage may be automatically adjusted in an ion source or a collision energy (that is applied to ions in order to cause fragmentation) may be adjusted with regard to collision cell operation. Such automatic instrument adjustments may be performed, for instance, so as to optimize the type or number of ions or ion fragments produced.

In Step **242** of the method **240** (FIG. 10A), one or more elution events of compounds within a current region of interest (ROI) are detected. The one or more elution events may be detected as peaks within within individual scans or within an ion chromatogram, such as an extracted ion chromatogram (XIC). In some embodiments, the ion chromatogram may be directly measured and provided by the analytical instrument, perhaps as a measure of total ion current versus time. The ion chromatogram provided by the analytical instrument may relate only to detection of precursor ions. Alternatively, a second ion chromatogram relating to product or fragment ions may also be provided by the analytical instrument. As a still further alternative, the instrument may simply provide raw data in the form of a series of mass spectra, each mass spectrum ("scan") relating to a certain measurement time and comprising intensity data relating to the detection of possibly many different ion masses, such as, for example, precursor ion masses within a certain experimental range of masses. In such cases, the one or more ion chromatograms may be simply calculated in Step **242** by digitally adding together the intensities of the various detected peaks in each scan or by extracting time-varying data in one or more mass ranges (such as extracted ion chromatograms or XICs) by considering variations between multiple individual scans.

The peaks in an ion chromatogram may be detected by the methods of Parameterless Peak Detection as taught in U.S. Patent No. 7,983,852 assigned to the assignee of the instant invention and incorporated herein in its entirety. In some instances, the region of interest may be defined as a time region around a single detected peak or envelope of peaks - such as, for instance, a time region bounded by limits that are at a distance of twice the standard deviation from a peak maximum on either side of the peak maximum. In some instances, the region of interest may be known or may be estimated prior to performing a particular analysis and may relate to an expected retention time of an expected or target analyte.

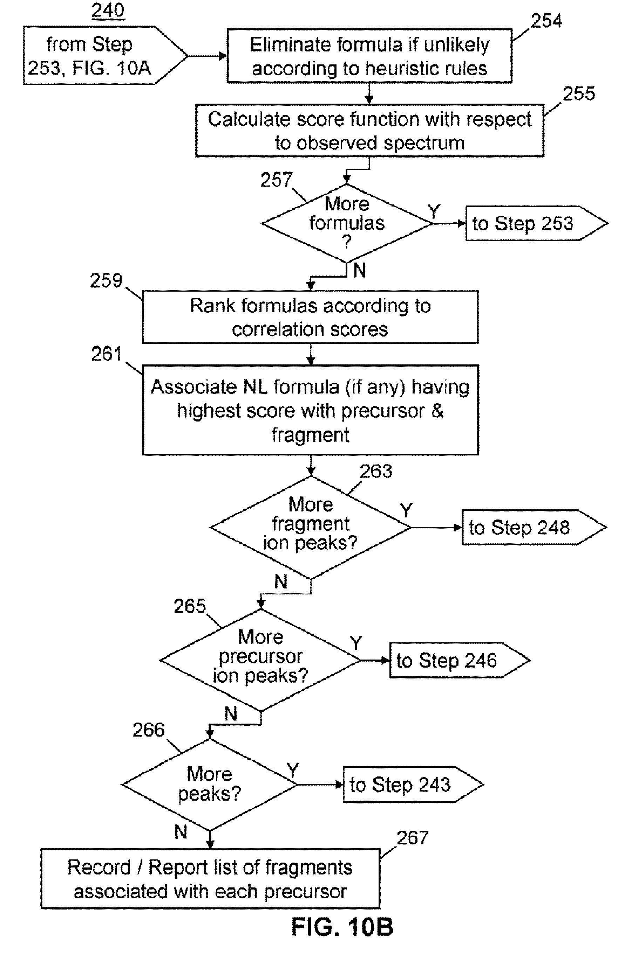

In the subsequent Step **243,** the first such identified peak is selected and subsequently considered in a loop of steps spanning from Step **243** to Step **266** (FIG. 10B). In Steps **244** and **245,** precursor-ion and fragment-ion peaks, respectively, are identified. The precursor-ion and product-ion or fragment-ion peaks may be identified by calculating extracted ion chromatograms as discussed previously in this document, each such ion chromatogram providing a representation of the quantity of ions detected within a respective mass range versus time. Each peak identified in either Step **244** or Step **245** represents a respective mass-to-charge range of ions whose detected intensity rises and falls in correspondence to a particular retention time.

In Step **246** of the method **240,** a first precursor ion peak - as identified in Step **244 -** is selected for consideration within a loop of steps spanning from Step **246** (FIG. 10A) to Step **265** (FIG. 10B). In Step **247,** the charge state and mass of the precursor ion peak under consideration is determined. The charge state may be determined by the spacing between the various peaks of an isotopic distribution of peaks, provided that the instrumental resolution is sufficient. With the magnitude of the charge thus known, the mass of the ion may be thus determined. In Step **248,** a first fragment-ion peak - as identified in Step **245 -** is selected for consideration within a loop of steps spanning from Step **248** (FIG. 10A) to Step **263** (FIG. 10B).

In Step **249,** the charge state and mass of the fragment-ion peak under consideration is determined. The charge state may be determined by the spacing between the various peaks of an isotopic distribution of peaks, provided that the instrumental resolution is sufficient. With the magnitude of the charge thus known, the mass of the ion may be thus determined. Generally, the fragment ion generated by neutral loss should comprise the same charge number as the precursor from which it was formed, the only exceptions being in special cases involving charge transfer. However, assuming collision-induced-dissociation fragmentation not including charge transfer in the dissociation mechanism, then the decision Step **250** is executed. If, in Step **250,** the fragment ion does not comprise the same charge number, then the next identified fragment ion peak is considered (Step **248)** as indicated by the dashed arrow in FIG. 10A. Otherwise, if the two charge numbers are the same, then Step **251** is executed.

In Step **251,** the mass of the fragment ion currently under consideration is subtracted from the mass of the precursor ion currently under consideration so as to provide a tentative mass difference. A list of candidate neutral loss (NL) formulas corresponding to the tentative mass difference is calculated or determined from a table of formula masses in Step **252.** Subsequently, in Step **253,** the first candidate neutral loss formula is considered. Note that the candidate formulas do not correspond directly to observed masses but, instead, to calculated mass differences between candidate precursor and product ions.

The candidate formula under consideration may, in some embodiments, be eliminated in Step **254** if it is deemed to be unlikely or unrealistic according to various heuristic rules. A list of such rules has been set forth by Kind and Fiehn ("Metabolomic database annotations via query of elemental compositions: Mass accuracy is insufficient even at less than 1 ppm", BMC Bioinformatics 2006, 7:234; "Seven Golden Rules for heuristic filtering of molecular formulas obtained by accurate mass spectrometry", BMC Bioinformatics 2007, 8:105). According to Kind and Fiehn, high mass accuracy (1 ppm or better) and high resolving power are desirable but insufficient for correct molecule identification. With regard to the method **240,** mass precision is a relevant quantity since, according to the methods taught herein, lists of tentative neutral loss molecules are derived by subtracting product-ion masses from precursor-ion masses. With regard to the present teachings, therefore, mass precision of 1 ppm or better is desirable. Such mass precision is available on commercially available electrostatic trap mass spectrometer systems (e.g., Orbitrap® mass spectrometer systems) as well as on time-of-flight (TOF) and other mass spectrometer systems. However, according to Kind and Fiehn, in order to eliminate ambiguities in formula assignments, certain molecules must either be eliminated or determined to be unlikely based on certain rules.

The rules set forth by Kind and Fiehn include a restriction rule relating to the number-of-elements, the LEWIS and SENIOR chemical rules, a rule relating to hydrogen/carbon ratios, a rule relating to the element ratio of nitrogen, oxygen, phosphor, and sulphur versus carbon, a rule relating to element ratio probabilities and a rule relating to the presence of trimethylsilylated compounds. For small organic molecules, such as drugs or their metabolites, the number of elements may be restricted to just the most common elements (e.g., C, H, N, S, O, P, Br and Cl and, possibly Si for some compounds that have been derivitized) and the numbers for nitrogen, phosphor, sulphur, bromine and chlorine should be relatively small relative to carbon. Further, the hydrogen/carbon ratio should not exceed approximately H/C > 3. According to the LEWIS rule, carbon, nitrogen and oxygen are expected to have an "octet" of completely filled s, p-valence shells. The SENIOR rule relates to the required sums of valences.

Some of the Kind and Fiehn rules (for example, valence rules) may be used to positively exclude certain molecules. Others of the rules may be used to calculate likelihoods or probabilities of occurrences based on tabulated observations of large collections of molecular formulas. For example, Kind and Fiehn (2007) present a histogram of hydrogen/carbon ratios for 42,000 diverse organic molecules which may be approximated by a probability density function. Probability density functions - either symmetric or skewed - may be similarly generated with regard to other element ratios. A candidate molecular formula may thus be compared against the various probability functions resulting from application of several of the heuristic rules and assigned a respective likelihood score based on each such rule. As further set forth by Kind and Fiehn, likelihood score may also be calculated in terms of the degree of matching or correlation between theoretical and observed isotopic patterns. In the present case, there is no directly observable isotopic pattern, because the candidate molecules all represent possible losses of neutral molecules. However, a pattern may be generated indirectly by conducting additional operations, in Step **251,** of normalizing the intensities of the observed isotopic distribution patterns of both candidate precursor and product molecules to their respective monoisotopic masses, shifting the mass axes such that monoisotopic masses overlap and then performing a simple spectral subtraction. An isotopic match score may be calculated based on a measure of correlation between the molecular isotopic pattern so calculated and an expected isotopic pattern of a candidate molecular formula.

A respective value of a formula score function is calculated in Step **255,** for those formulas that are not eliminated in Step **254.** In some embodiments, the overall formula score function may be calculated as a product of the individual likelihood scores or correlation scores calculated by application of the individual likelihood rules discussed above. The formulas which are positively excluded by certain of the rules may be eliminated from consideration in Step **254,** prior to this calculation. Alternatively, such excluded formulas may be presumed to comprise scores which are calculated including at least one factor which is equal to zero. In some embodiments, most of the rules may be formulated so as to yield a simple binary "yes" or "no" answer regarding the exclusion of or possible allowance of a certain formula. The final likelihood score for formulas which are not excluded in this fashion may be then calculated from the isotopic correlation scores.

Then, in the loop termination step, Step **257** (FIG. 10B), if there are additional candidate neutral loss formulas to be considered, execution of the method **240** returns to Step **253** and the next candidate neutral loss formula in the list is considered, in turn. Once the value of the formula score function has been calculated for all candidate neutral loss formulas, the various formulas are ranked according to their scores in Step **259.**

In Step **261,** the candidate neutral loss formula (if any) having the highest score may be associated with the precursor ion and fragment ion currently under consideration. However, if there are no candidate neutral loss formulas whose scores are at or above a pre-determined threshold, then no such formula is associated with the precursor ion and fragment ion. The assignment of a neutral loss formula to a precursor-product pair indicates that there is a significant probability that the fragment ion under consideration is related to the precursor ion under consideration by fragmentation of the precursor such that a neutral molecule having the assigned formula is released at the time of formation of the fragment ion.

In the loop termination step, Step **263,** if there are additional fragment-ion peaks within the ROI that have not been considered in conjunction with the precursor ion currently under consideration, then execution of the method **240** returns to Step **248** (FIG. 10A) and the next identified fragment-ion peak is considered, in turn. Otherwise, execution proceeds to the next loop termination step, Step **265.** If, in Step **265,** there are additional precursor-ion peaks within the ROI that have not been considered, then execution of the method **240** returns to Step **246** (FIG. 10A) and the next identified precursor-ion peak is considered, in turn. Otherwise, execution proceeds to the next loop termination step, Step **266.** If, in Step **266,** there are additional chromatogram peaks or elution events that have not been considered, then execution of the method **240** returns to Step **243** (FIG. 10A) and the next identified elution event or peak is considered, in turn. Otherwise, execution proceeds to the final step, Step **267,** of the method, in which a list of related precursor-fragment pairs, as determined by the values of the formula score function, is reported or stored.

The results are stored for later use (and possibly reported to a user) in Step **267.** The results may include calculated product/precursor matches, information regarding detected peaks or other information. Recorded or reported peak parameters may be either those parameters calculated during the peak detection step or quantities calculated from those parameters and may include, for each of one or more peaks, location of peak centroid, location of point of maximum intensity, peak half-width, peak skew, peak maximum intensity, area under the peak, etc. Other parameters related to signal to noise ratio, statistical confidence in the results, goodness of fit, etc. may also be recorded / reported in Step **267.**

Spectral Recognition and Library Updating

In various embodiments, decomposed reconstructed MS-2 spectra may be written to a database when there are at least a certain number of product masses (or mass-to-charge ratios) in the reconstructed MS-2 spectrum. In some embodiments, each entry in the local mass spectral library may comprise a list of the mass-to-charge ratios (*m/z* values) observed in a previously-observed reconstructed MS-2 spectrum. In some embodiments, one or more entries may also include a mass-to-charge ratio of a precursor ion from which the ions in the MS-2 spectrum of the respective entry were derived. In some embodiments, one or more entries may also include a value of a chromatographic retention time at which a precursor ion or the ions in the MS-2 spectrum of the respective entry were observed. In some embodiments, one or more entries may also include an identification of a compound from which a precursor ion or the MS-2 spectra of the respective entry were derived. In some embodiments, one or more entries may also include an annotation or comment regarding the nature of a compound or of the mass spectra of the respective entry. Such comments may be incorporated into the local mass spectral library by a trained user upon reviewing the data.

In a sample of 10 typical raw files (average size 57 MB) generated by an Exactive™ mass spectrometer, 2785 such spectra were found using a threshold requirement of at least four product ion masses in the MS-2 spectrum. This number represents, on average, about 280 spectra per data file or approximately 25% of the total number of components found. If desired, the threshold number of MS-2 *m/z* values required to recognize or to store a spectrum could be adjusted, giving either fewer or more database spectra. This value of 280 spectra/file corresponds to a data compression from 570 MB to approximately 300 KB (i.e., a compression of more than 1000: 1), so months worth of data can be stored, but more importantly, this data can easily and quickly be searched for a match. Although this exemplary analysis utilized a requirement of finding a threshold of at least four product ion masses, any number greater than zero may be employed as a threshold number of product ion masses.

Other types of data may be more component-rich. Other data files have been examined that yield five times the number of components as the data mentioned above (when measured as the number of valid MS-2 spectra per MB of file size), but that still produces a compression of almost 500:1.

Both of these example compression ratios assume that every component found is interesting. In reality, however, a majority of the components will comprise known contaminants or solvent peaks. By running blank samples, and automatically generating a database of blank MS-2 spectra, many of the recurring spectra could be identified as background. Or, recurring matches could be reviewed by a spectrometrist and flagged as either known or uninteresting compounds, or as compounds of interest.

The decomposed reconstructed MS-2 spectra generated according to the present teachings may be compared to entries in the local mass spectral library corresponding compounds previously measured using the same mass spectrometer. In some embodiments, the matched data, if not associated with a compound identification in the local spectral library, could be searched against a curated database of known compounds to identify the actual compound present. However, in many cases, it may be sufficient to learn that the detected compounds, while not identified exactly, were found in previous samples. This corresponds to a report that the compound in question was found at a certain retention time in a certain sample run on a previous date. It has been found that, when data is processed after acquisition, a search of a 100 MB database file takes only a few tens of milliseconds per query record. However to achieve all the benefits of this invention, the processing and database search would be done by the instrument as the data is collected. In such cases, such real-time processing could be employed so as to make automated real-time decisions about the course of subsequent mass spectral scans on a single sample or during a single chromatographic separation. Such decisions could include, for example, variation of instrumental operating parameters such as, for example, collision energy level.

Conclusions

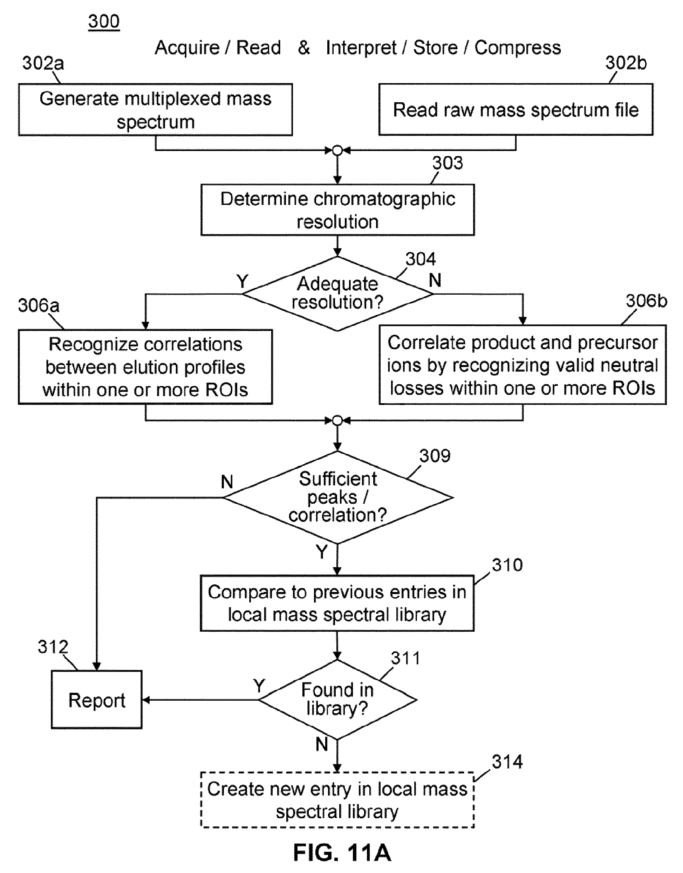

FIG. 11A is a flow chart of a first general method in accordance with the present teachings for acquiring or inputting mass spectral data, interpreting that data in terms of a mass spectral library, and, optionally storing the data, possibly in a compressed format. The general method **300** illustrated in FIG. 11A could be considered to encompass several more-specific methods, depending on the branches followed through the flowchart or the decision to either execute or not execute the steps illustrated in outlined boxes. In one of these more-specific aspects, the method **300** could be considered as a method for acquiring data using a mass spectrometer system and interpreting that data, as it is acquired, in terms of spectra previously observed using the system and recorded in a local mass spectral library. In another aspect, the method **300** could be considered as a method or reading mass spectral data and then interpreting or storing the data or compressing the file size of stored data. As a still-further alternative, the method **300** could be considered as a method for compressing the file size of a computer data file previously generated using the mass spectrometer system so as to transform the file into (or replace the file by) a local mass spectral library.

Execution of the method **300** may begin at either Step **302a** if data is being either interpreted or stored as it is acquired or at Step **302b** if data is being read from data previously stored in a raw data file. Accordingly, in Step **302a,** multiplexed mass spectral data is generated by the mass spectrometer system; in Step **302b,** date relating to previously generated multiplexed mass spectra are read or inputted from a data file or from a data storage device. In Step 303, the chromatographic resolution of the data is determined. Subsequent Step **304** is a branching step, with the direction of branching being determined with regard to whether the chromatographic resolution of the data is adequate to generate sufficiently resolved intensity-versus-time profiles of mass spectral peaks (extracted ion chromatograms) so as to enable recognition of overlapped elution profiles. In practice, the adequacy may be related to whether there exists a threshold number of scans across the chromatographic peaks in a region of interest in question. For example, if there are at least 7-9 scans across each chromatographic peak then Step **306a** may be executed whereas, if there are fewer scans across some peaks, then Step **306b** may be executed.

If the chromatographic resolution is determined to be adequate in Step **304,** the Step **306a** is executed, in which correlations between elution profiles are recognized, for instance, by employing the method **80** (FIG. 5) and the method **40** (FIG. 6) or some similar method. Otherwise, Step **306b** is executed, in which correlations between product and precursor ions are recognized by recognizing valid neutral losses, for example, by employing the method **240** (FIG. 10) or some similar method.

The methods employed in either the Step **306a** or the Step **306b** are designed to automatically identify mass spectral peaks of both precursor ions and product or fragment ions and to subsequently identify likely possible precursor-product relationships within the data by attempting to recognize correlations among the identified peaks, as described previously herein. Depending upon the quality of the data or the nature or condition of the sample, such identifications and recognitions may or may not be successful. Therefore, a test is made, in the Step **309** to determine if spectral peaks are adequately identified and characterized, or if a sufficient number of peaks are identified, or if recognized correlations are reliable, or if a sufficient number of correlations are recognized. Information used in this step may include, without limitation the values of identified peak parameters, spectral noise levels, and correlation scores. These values may be compared to various pre-determined thresholds in Step **309** in order to assess the reliability of identified peaks and recognized correlations. If the results are determined to be reliable, then Step **310** is then executed, in which the identified and recognized information are compared to information previously stored in a local mass spectral library, as previously described herein.

Execution of the method **300** may stop at the reporting Step **312** if the quality or number of the peak identifications or correlations are judged to be inadequate or if the acquired data is simply being compared to information in the local mass spectral library, possibly for purposes of identifying an analyte. However, if the execution of the Step **306a** or the Step **306b** results in recognition of spectral data that was not previously recorded in the local mass spectral library (Step **311)** and if the new spectral data comprises a sufficient number of spectral peaks and correlations of adequate quality determined to be necessary to recognize new data (Step **309),** then a new entry may be made in the local mass spectral library (Step **314).** Step **314** will be executed any time that data from a raw file is being read (Step **302b)** and stored to a local mass spectral library for purposes of file size compression. Step **314** may also be executed - although not necessarily executed - in cases in which data is being analyzed as it is being acquired by a mass spectrometer system.

The mass spectral library may be partitioned into sub-libraries or may comprise separate individual libraries corresponding to different classes of data or samples. For example, the mass spectral library may comprise two individual libraries or partitions with a first partition containing data relating to analytes of interest and a second partition containing data relating to common solvent or other chemical components which may be expected to be present in chromatographic fluids. The data of the second such partition or library may be developed by running "blank" samples which contain only the solvents and other compounds (e.g., pH buffer compounds) which are normally present during chromatographic experiments. In this way, non-analyte materials may be readily recognized so as to prevent the making of non-diagnostic entries into the analyte partition or analyte library.

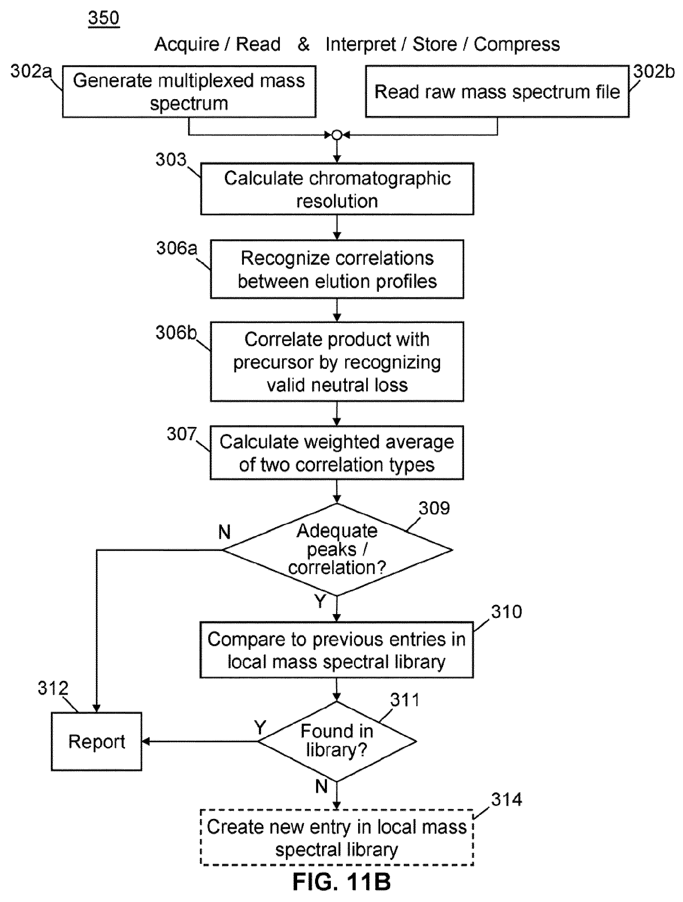

FIG. 11B shows a flow chart of a second general method in accordance with the present teachings for acquiring or inputting mass spectral data, interpreting that data in terms of a mass spectral library, and, optionally storing the data, possibly in a compressed format. The general method **350** illustrated in FIG. 11A is similar to the method 300 shown in FIG. 11A except that Steps **306a** and **306b** are both executed and, afterwards, in Step **307,** a weighted average of correlation scores determined in both steps is calculated. In this method, two separate analyses are performed using the same data set - a first analysis using the method **80** (which employs the method **40)** and a second analysis using the method **240.** To avoid duplication of calculation steps and thereby increase calculation speed, the peak identifications may be made only one time and then employed in the two different correlation calculations - a first correlation score calculation based on peak profiles and a second correlation score calculation based on recognition of valid neutral losses. The weighting scheme employed in Step **307** may be based on the chromatographic resolution determination made in Step **303.** That is, better chromatographic separation and/or greater numbers of mass spectral scans across chromatographic peaks will generally lead to greater weight being given to the elution profile correlation method and vice versa.

The novel methods provided herein are able to create high quality noise-free MS-2 spectra suitable for archiving in a database, for reference use against subsequent experiments. Since the disclosed methods do not rely on any user-adjustable parameters, these comparisons may be done by the instrument as the data is being collected, in order to modify an experiment based on the presence or absence of compounds of interest. The analyses taught herein may also or alternatively be performed on archival data that has not previously been analyzed in this manner, or that has not been analyzed against a subsequently created database of compounds. This allows new information to be gleaned from existing data without the requirement of repeating experiments. By means of periodic review of the recurring spectral matches, by a trained spectrometrist, compounds that come from known impurities, or from solvents, could be marked as uninteresting, and compounds that are known but relevant could also be marked, improving the automatic compound recognition overtime. These annotations may be entered directly into the entries corresponding to the respective spectra or compounds.

The discussion included in this application is intended to serve as a basic description. Although the invention has been described in accordance with the various embodiments shown and described, one of ordinary skill in the art will readily recognize that there could be variations to the embodiments and those variations would be within the spirit and scope of the present invention. The reader should be aware that the specific discussion may not explicitly describe all embodiments possible; many alternatives are implicit. Accordingly, many modifications may be made by one of ordinary skill in the art without departing from the spirit, scope and essence of the invention. Neither the description nor the terminology is intended to limit the scope of the invention. Any patents, patent applications, patent application publications or other literature mentioned herein are hereby incorporated by reference herein in their respective entirety as if fully set forth herein except that, insofar as such patents, patent applications, patent application publications or other literature may conflict with the present specification, then the present specification will control.

**Claims**

1. A method of acquiring and interpreting data using (i) a mass spectrometer system and (ii) a mass spectral library having a plurality of library entries derived from data previously obtained using said mass spectrometer system, said method comprising:

   (a) generating a multiplexed mass spectrum using the mass spectrometer system, the multiplexed mass spectrum comprising a superposition of a plurality of product-ion mass spectra comprising a plurality of product-ion types having respective product-ion mass-to-charge ($m/z$) ratios, each product-ion mass spectrum corresponding to fragmentation of a respective precursor-ion type formed by ionization of a chemical compound, each precursor-ion type having a respective precursor-ion $m/z$ ratio;
   (b) recognizing a respective set of product-ion types corresponding to each of one or more of the product-ion mass spectra by recognizing correlations between the elution profiles of said product-ion types of each said respective set; and
   (c) determining if each recognized set of product-ion types corresponds to a product-ion mass spectrum previously observed using said mass spectrometer system by comparing the $m/z$ ratios of the product ion types of each said recognized set to information in at least one entry of the mass spectral library.

2. A method as recited in claim 1, further comprising, if a recognized set of product-ion types is determined to not correspond to any product-ion mass spectrum previously observed using said mass spectrometer system:

   (d) creating a new entry in the mass spectral library, said new entry including said recognized set of two or more product ion types.

3. A method as recited in claim 1, further comprising, if a recognized set of product-ion types is determined to not correspond to any product-ion mass spectrum previously observed using said mass spectrometer system:

   (d) determining an identity of a chemical compound corresponding to said recognized set of two or more product-ion types by comparing the $m/z$ ratios of the product ions of each said recognized set to a database of sets of product-ion $m/z$ ratios corresponding to respective chemical compounds; and
   (e) creating a new entry in the mass spectral library, said new entry including said recognized set of two or more product ion types and the determined chemical compound identity.

4. A method as recited in claim 1, wherein the step (b) of recognizing a respective set of product-ion types corresponding to each of one or more of the product-ion mass spectra comprises recognizing said each respective set of product-ion types and recognizing a respective precursor-ion type corresponding to each of the one or more of the product-ion mass spectra, the recognizing performed by recognizing correlations between the elution profiles of the product-ion types and the precursor-ion type corresponding to each of the one or more of the product-ion mass spectra.

5. A method as recited in claim 1, wherein the step (c) of determining if each recognized set of product-ion types corresponds to a product-ion mass spectrum previously observed using said mass spectrometer system includes determining if each recognized set of product-ion types corresponds to a chemical compound previously introduced into the mass spectrometer system.

6. A method as recited in claim 1, wherein the recognizing of correlations between the elution profiles of said product-ion types corresponding to each said respective set comprises:

   choosing a time window defining a region of interest for experimental data relating to the product-ion types generated by the mass spectrometer system;
   constructing a plurality of extracted ion chromatograms (XICs) for the experimental data relating to the product-ion types within the region of interest;
   automatically detecting and characterizing chromatogram peaks within each XIC and automatically generating synthetic analytical fit peaks thereof;
   discarding a subset of the synthetic analytical peaks which do not satisfy noise reduction rules;

performing a respective cross-correlation score calculation between each pair of synthetic analytical fit peaks; and

recognizing said correlations between the elution profiles of said product-ion types corresponding to each said respective set based on the cross correlation scores.

7. A method of acquiring and interpreting data using (i) a mass spectrometer system and (ii) a mass spectral library having a plurality of library entries derived from data previously obtained using said mass spectrometer system, said method comprising:

(a) generating a multiplexed mass spectrum using the mass spectrometer system, the multiplexed mass spectrum comprising a superposition of a plurality of product-ion mass spectra comprising a plurality of product-ion types having respective product-ion mass-to-charge (*m/z*) ratios, each product-ion mass spectrum corresponding to fragmentation of a respective precursor-ion type formed by ionization of a chemical compound, each precursor-ion type having a respective precursor-ion mass-to-charge (*m/z*) ratio;

(b) recognizing a set comprising a precursor-ion type and one or more product-ion types corresponding to each of one or more product-ion mass spectra by recognizing one or more losses of a respective valid neutral molecule from each said precursor-ion type; and

(c) determining if each recognized set of a precursor-ion type and one or more product-ion types corresponds to a compound whose mass spectra were previously observed using said mass spectrometer system by comparing the *m/z* ratios of said precursor-ion type and said one or more product ion types of each said recognized set to information in at least one entry of the mass spectral library.

8. A method as recited in claim 7, further comprising, if a recognized set of a precursor ion type and one or more product-ion types is determined to not correspond to any compound whose mass spectra were previously observed using said mass spectrometer system

(d) creating a new entry in the mass spectral library, said new entry including said recognized set of two or more product ion types.

9. A method as recited in claim 7, wherein the recognizing of one or more losses of a respective valid neutral molecule from each said precursor-ion type comprises:

(b1) determining the charge state and mass of each said precursor-ion type;

(b2) determining the charge state and mass of each of the plurality of product-ion types;

(b3) subtracting the mass of each of the plurality of product-ion types from the mass of each said precursor-ion type so as to generate a list of tentative molecular masses for each said precursor-ion type;

(b4) tabulating a list of tentative molecular formulas for each tentative molecular mass;

(b5) ranking each list of tentative molecular formulas according to chemical likelihood rules and an isotopic pattern correspondence;

(b6) assigning the highest-ranked tentative molecular formula to its respective tentative molecular mass if the ranking of the highest-ranked tentative molecular formula exceeds a threshold value; and

(b7) for each pair of precursor-ion type and product-ion type corresponding to a tentative molecular mass corresponding to an assigned tentative molecular formula, recognizing the assigned tentative molecular formula as a loss of a valid neutral molecule.

10. A method of reducing a size of a computer file of mass spectral data obtained with regard to a sample using a mass spectrometer system, said mass spectral data comprising a plurality of multiplexed mass spectra obtained at respective elution times, wherein each said multiplexed mass spectrum comprises a superposition of a plurality of product-ion mass spectra comprising a plurality of product-ion types, each product-ion mass spectrum corresponding to fragmentation of a respective precursor-ion type formed by ionization of a chemical compound of the sample, each precursor-ion type having a respective precursor-ion mass-to-charge (*m/z*) ratio and each product ion type having a respective product-ion *m/z* ratio, said method comprising:

(a) extracting a respective elution profile of each product-ion type;

(b) calculating a respective correlation score between each possible pair of extracted elution profiles;

(c) recognizing sets of correlated product-ion types such that the calculated correlation scores between each pair of product-ion types of the set is above a threshold correlation score; and

(d) retaining information within the computer file only in regard to those recognized sets for which the number of correlated product-ion types of the set is above a threshold number of product-ion types.

11. A method of reducing a size of a computer file of mass spectral data obtained with regard to a sample using a mass spectrometer system, said mass spectral data comprising a plurality of multiplexed mass spectra obtained at respective elution times, wherein each said multiplexed mass spectrum comprises a superposition of a plurality of product-ion mass spectra comprising a plurality of product-ion types, each product-ion mass spectrum corresponding to fragmentation of a respective precursor-ion type formed by ionization of a chemical compound of the sample, each precursor-ion type having a respective precursor-ion mass-to-charge ($m/z$) ratio and each product ion type having a respective product-ion $m/z$ ratio, said method comprising:

    (a) recognizing a plurality of sets, each set comprising a precursor-ion type and one or more product-ion types such that each product-ion type of each set corresponds to a loss of a respective valid neutral molecule from the precursor-ion type of said each set; and
    (d) retaining information within the computer file only in regard to those recognized sets for which the number of product-ion types of the set is above a threshold number of product-ion types.

12. A method of acquiring and interpreting data using (i) a mass spectrometer system and (ii) a mass spectral library having a plurality of library entries derived from data previously obtained using said mass spectrometer system, said method comprising:

    (a) generating a multiplexed mass spectrum using the mass spectrometer system, the multiplexed mass spectrum comprising a superposition of a plurality of product-ion mass spectra comprising a plurality of product-ion types having respective product-ion mass-to-charge ($m/z$) ratios, each product-ion mass spectrum corresponding to fragmentation of a respective precursor-ion type formed by ionization of a chemical compound, each precursor-ion type having a respective precursor-ion mass-to-charge ($m/z$) ratio;
    (b) identifying a precursor-ion type and a set comprising one or more tentative product-ion types by calculating, for each respective tentative product-ion type, a neutral-loss correlation score corresponding to a likelihood that said each respective tentative product-ion type is the result of a loss of a valid neutral molecule from the precursor-ion type;
    (c) calculating a respective profile correlation score between the elution profile of the precursor-ion type and each said tentative product-ion type;
    (d) calculating a weighted average value between the neutral-loss correlation score and the profile correlation score corresponding to each tentative product-ion type;
    (e) recognizing one or more of the tentative product-ion types as being related to the precursor-ion type by fragmentation thereof, based on the calculated weighted values; and
    (f) determining if the precursor-ion type and the one or more recognized related product-ion types corresponds to a compound whose mass spectra were previously observed using said mass spectrometer system by comparing the $m/z$ ratios of said precursor-ion type and said one or more recognized related product ion types to information in at least one entry of the mass spectral library.

13. A method as recited in claim 12, wherein weighting factors employed in the calculating of the weighted average values are determined based on a chromatographic resolution of a chromatograph that supplies samples to the mass spectrometer.

14. A method of acquiring and interpreting data using (i) a mass spectrometer system and (ii) a mass spectral library having a plurality of library entries derived from data previously obtained using said mass spectrometer system, said method comprising:

    (a) generating a multiplexed mass spectrum using the mass spectrometer system, the multiplexed mass spectrum comprising a superposition of a plurality of product-ion mass spectra comprising a plurality of product-ion types having respective product-ion mass-to-charge ($m/z$) ratios, each product-ion mass spectrum corresponding to fragmentation of a respective precursor-ion type formed by ionization of a chemical compound, each precursor-ion type having a respective precursor-ion mass-to-charge ($m/z$) ratio;
    (b) identifying a precursor-ion type and a set comprising one or more tentative product-ion types by calculating, for each respective tentative product-ion type, a profile correlation score between the elution profile of the precursor-ion type and said each tentative product-ion type;
    (c) calculating, for each respective tentative product-ion type comprising an identified charge state that is identical to an identified charge state of said precursor-ion type, a neutral-loss correlation score corresponding to a likelihood that each respective tentative product-ion type is the result of a loss of a valid neutral molecule from said precursor-ion type;

(d) calculating, for each respective tentative product-ion type comprising the identified charge state that is identical to the identified charge state of said precursor-ion type, a weighted average value between the neutral-loss correlation score and the profile correlation score corresponding to each tentative product-ion type;

(e) recognizing one or more of the tentative product-ion types as being related to the precursor-ion type by fragmentation thereof, based on the calculated weighted values; and

(f) determining if the precursor-ion type and the one or more recognized related product-ion types corresponds to a compound whose mass spectra were previously observed using said mass spectrometer system by comparing the $m/z$ ratios of said precursor-ion type and said one or more recognized related product ion types to information in at least one entry of the mass spectral library.

15. A method as recited in claim 14, wherein weighting factors employed in the calculating of the weighted average values are determined based on a chromatographic resolution of a chromatograph that supplies samples to the mass spectrometer.

FIG. 1
(Prior Art)

EP 2 741 225 A2

10

33      34      35

Chromatograph | Mass Spectrometer [1] [39] | Detector

32

Sample

37     38

36

Programmable Processor | Output / Display

Electronic Data Storage

**FIG. 2**

24

FIG. 3

**FIG. 4A**

26

**FIG. 4B**

**FIG. 4C**

<u>80</u>

```
Obtain mass spectral data                          81

Select first or next region of
interest (ROI) of data                             83

Execute method 40 (FIG. 6)
with regard to ROI                                 85

                    Last ROI in        87
            N       data set?
                         │ Y

                    Any                89
            N       prevalent
                    m/z values?
                         │ Y

Execute method 40 (FIG. 6)
using full time range as ROI                       91

Calculate cross-
correlation scores                                 93

Report / store
results                                            95
```

**FIG. 5**

FIG. 6A

40

from Step
49, FIG. 6A

Current peak = first XIC peak    51

52
Y ← Area / intensity
rules satisfied? → N

Discard peak
from cross-
correlation
candidates    53

Retain peak parameters for
cross correlation    54

Subtract peak area
from XIC area total    55

Add peak to list of
considered peaks    56

More peaks
in XIC?    57
N →
Y

Any m/z
values in
all scans?    58
N →
Y

Add such m/z values
to list of prevalent
m/z values    59

Current peak =
next XIC peak    60

to Step 44,
FIG. 6A

**FIG. 6B**

48

Detect and characterize peak components
in chromatogram using PPD

Remove baseline and
estimate noise level

120

Detect peaks and
estimate peak parameters

150

Optionally, improve estimated
coefficient values by simultaneously
optimizing overlapping peaks

170

**FIG. 7A**

Intensity
(arbitrary units)

103

104      105

Retention time

**FIG. 7B**

FIG. 8

FIG. 9

<u>240</u>

241
Start: Receive all-ions data in ROI

242
Detect / list peaks in data in ROI

243
Select first (or next) identified elution event ← from Step 266

244
Identify precursor ion peaks

245
Identify fragment ion peaks

246
Select first (or next) identified precursor ion peak ← from Step 265

247
Determine precursor charge & mass

248
Select first (or next) identified fragment ion peak ← from Step 263

249
Determine fragment charge & mass

250
Same charge?
N
Y

251
Subtract fragment mass from precursor mass

252
Calculate or tabulate list of candidate neutral loss (NL) formulas corresponding to mass

253
Select first (or next) NL formula ← from Step 257

to Step 254, FIG. 10B

**FIG. 10A**

240

from Step
253, FIG. 10A

Eliminate formula if unlikely
according to heuristic rules 254

Calculate score function with respect
to observed spectrum 255

257 More
formulas
? — Y → to Step 253

N

259 Rank formulas according to
correlation scores

261 Associate NL formula (if any) having
highest score with precursor &
fragment

263 More
fragment
ion peaks? — Y → to Step 248

N

265 More
precursor
ion peaks? — Y → to Step 246

N

266 More
peaks? — Y → to Step 243

N

Record / Report list of fragments
associated with each precursor 267

**FIG. 10B**

**FIG. 11A**

350

Acquire / Read & Interpret / Store / Compress

302a — Generate multiplexed mass spectrum

302b — Read raw mass spectrum file

303 — Calculate chromatographic resolution

306a — Recognize correlations between elution profiles

306b — Correlate product with precursor by recognizing valid neutral loss

307 — Calculate weighted average of two correlation types

309 — Adequate peaks / correlation?

N

Y

310 — Compare to previous entries in local mass spectral library

311 — Found in library?

Y

312 — Report

N

314 — Create new entry in local mass spectral library

**FIG. 11B**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 02078046 A
- US 5886346 A
- US 6872938 B
- US 12970570 B
- US 20120158318 A1
- US 7983852 B